# EUROPEAN PATENT SPECIFICATION

(11) **EP 3 156 411 B1**
(45) Date of publication and mention of the grant of the patent: **03.03.2021**
(21) Application number: 15803039.5
(22) Date of filing: 04.06.2015
(51) Int. Cl.: C07K 7/00, A61K 31/16, A61K 31/4409, A61K 38/00, A61K 47/50, A61P 35/00, A61P 43/00, C12N 15/09, A61K 47/64, A61K 47/55, A61K 47/54

(54) **ARTIFICIAL CATALYST SYSTEM CAPABLE OF SUBSTITUTING FOR IN VIVO ACYLATION FUNCTION**
ZUR ERSETZUNG VON IN-VIVO-ACYLIERUNGSFUNKTION FÄHIGES, KÜNSTLICHES KATALYSATORSYSTEM
SYSTÈME DE CATALYSEUR ARTIFICIEL CAPABLE DE REMPLACER UNE FONCTION D'ACYLATION IN VIVO

(30) Priority: 04.06.2014 JP 2014115494
(43) Date of publication of application: 19.04.2017
(73) Proprietor: The University of Tokyo, Tokyo 113-8654 (JP)
(72) Inventor: KANAI, Motomu, Tokyo 113-8654 (JP); KAWASHIMA, Shigehiro, Tokyo 113-8654 (JP); YAMATSUGU, Kenzo, Tokyo 113-8654 (JP); ZHU, Haiyan, Tokyo 113-8654 (JP); AMAMOTO, Yoshifumi, Tokyo 113-8654 (JP); TANABE, Kana, Tokyo 113-8654 (JP); ISHIGURO, Tadashi, Tokyo 113-8654 (JP); LIU, Jiaan, Tokyo 113-8654 (JP)
(74) Representative: J A Kemp LLP
(86) International application number: PCT/JP2015/066179
(87) International publication number: WO 2015/186785

(56) References cited:
- EP-A1- 1 424 328
- SHO-HEI FUJISHIMA ET AL: "Ligand-Directed Acyl Imidazole Chemistry for Labeling of Membrane-Bound Proteins on Live Cells", JOURNAL OF THE AMERICAN CHEMICAL SOCIETY, vol. 134, no. 9, 7 March 2012 (2012-03-07) , pages 3961-3964, XP55221548, US ISSN: 0002-7863, DOI: 10.1021/ja2108855
- HANGXIANG WANG ET AL: "Chemical Cell-Surface Receptor Engineering Using Affinity-Guided, Multivalent Organocatalysts", JOURNAL OF THE AMERICAN CHEMICAL SOCIETY, vol. 133, no. 31, 10 August 2011 (2011-08-10), pages 12220-12228, XP055238986, US ISSN: 0002-7863, DOI: 10.1021/ja204422r
- YOICHIRO KOSHI ET AL: "Target-Specific Chemical Acylation of Lectins by Ligand-Tethered DMAP Catalysts", JOURNAL OF THE AMERICAN CHEMICAL SOCIETY, vol. 130, no. 1, 1 January 2008 (2008-01-01), pages 245-251, XP55373199, US ISSN: 0002-7863, DOI: 10.1021/ja075684q
- TAKAHIRO HAYASHI ET AL: "Semisynthetic Lectin-4-Dimethylaminopyridine Conjugates for Labeling and Profiling Glycoproteins on Live Cell Surfaces", JOURNAL OF THE AMERICAN CHEMICAL SOCIETY, vol. 135, no. 33, 12 August 2013 (2013-08-12), pages 12252-12258, XP055429456, US ISSN: 0002-7863, DOI: 10.1021/ja4043214
- YOUSUKE TAKAOKA ET AL: "Protein Organic Chemistry and Applications for Labeling and Engineering in Live-Cell Systems", ANGEWANDTE CHEMIE INTERNATIONAL EDITION, vol. 52, no. 15, 20 February 2013 (2013-02-20), pages 4088-4106, XP055429550, ISSN: 1433-7851, DOI: 10.1002/anie.201207089
- HANGXIANG WANG ET AL.: 'Chemical Cell -Surface Receptor Engineering Using Affinity-Guided, Multivalent Organocatalysts' JOURNAL OF THE AMERICAN CHEMICAL SOCIETY vol. 133, no. 31, 2011, ISSN 0002-7863 pages 12220 - 12228, XP055238986
- YASUO KIKUGAWA ET AL.: 'N-METHOXYDIACETAMIDE: A NEW SELECTIVE ACETYLATING AGENT' TETRAHEDRON LETTERS vol. 31, no. 2, 1990, pages 243 - 246, XP055238989
- JAMES E. BROWNELL ET AL.: 'Tetrahymena Histone Acetyltransferase A: A Homolog to Yeast Gcn5p Linking Histone Acetylation to Gene Activation' CELL vol. 84, no. 6, 1996, ISSN 0092-8674 pages 843 - 851, XP000877462

### SEQUENCE LISTING

<110> THE UNIVERSITY OF TOKYO
<120> ARTIFICIAL CATALYST SYSTEM SUBSTITUTABLE FOR IN VIVO ACYLATION
   FUNCTION
<130> 140192001
<150> JP2014/115494
   <151> 2014-06-04
<160> 20
<170> PatentIn version 3.1
<210> 1
   <211> 21
   <212> DNA
   <213> Artificial
<220>
   <223> siRNA sequence: Combined DNA/RNA Molecule
<400> 1
   cggcacagcc ucucagucat t 21
<210> 2
   <211> 21
   <212> DNA
   <213> Artificial
<220>
   <223> siRNA sequence: Combined DNA/RNA Molecule
<400> 2
   ugacugagag gcugugccgt t 21
<210> 3
   <211> 23
   <212> DNA
   <213> Artificial
<220>
   <223> siRNA sequence: Combined DNA/RNA Molecule
<400> 3
   ugacacaggc aggcuugacu utt 23
<210> 4
   <211> 23
   <212> DNA
   <213> Artificial
<220>
   <223> siRNA sequence: Combined DNA/RNA Molecule
<400> 4
   aagucaagcc ugccuguguc att 23
<210> 5
   <211> 21
   <212> DNA
   <213> Artificial
<220>
   <223> siRNA sequence: Combined DNA/RNA Molecule
<400> 5
   gacuccagug guaaucuact t 21
<210> 6
   <211> 21
   <212> DNA
   <213> Artificial
<220>
   <223> siRNA sequence: Combined DNA/RNA Molecule
<400> 6
   guagauuacc acuggaguct t 21
<210> 7
   <211> 21
   <212> DNA
   <213> Artificial
<220>
   <223> siRNA sequence: Combined DNA/RNA Molecule
<400> 7
   cuacuuccug aaaacaacgt t 21
<210> 8
   <211> 21
   <212> DNA
   <213> Artificial
<220>
   <223> siRNA sequence: Combined DNA/RNA Molecule
<400> 8
   cguuguuuuc aggaaguagt t 21
<210> 9
   <211> 21
   <212> DNA
   <213> Artificial
<220>
   <223> siRNA sequence: Combined DNA/RNA Molecule
<400> 9
   cguacgcgga auacuucgat t 21
<210> 10
   <211> 21
   <212> DNA
   <213> Artificial
<220>
   <223> siRNA sequence: Combined DNA/RNA Molecule
<400> 10
   ucgaaguauu ccgcguacgt t 21
<210> 11
   <211> 21
   <212> DNA
   <213> Artificial
<220>
   <223> Artificially synthesized primer sequence
<400> 11
   cccaagcaat ggatgatttg a 21
<210> 12
   <211> 21
   <212> DNA
   <213> Artificial
<220>
   <223> Artificially synthesized primer sequence
<400> 12
   ggcattctgg gagcttcatc t 21
<210> 13
   <211> 21
   <212> DNA
   <213> Artificial
<220>
   <223> Artificially synthesized primer sequence
<400> 13
   gcgatggaac ttcgactttg t 21
<210> 14
   <211> 21
   <212> DNA
   <213> Artificial
<220>
   <223> Artificially synthesized primer sequence
<400> 14
   gggcttcctc ttggagaaga t 21
<210> 15
   <211> 20
   <212> DNA
   <213> Artificial
<220>
   <223> Artificially synthesized primer sequence
<400> 15
   agacggggtt agcggagcaa 20
<210> 16
   <211> 20
   <212> DNA
   <213> Artificial
<220>
   <223> Artificially synthesized primer sequence
<400> 16
   tcttgggcgt ctgctccaca 20
<210> 17
   <211> 20
   <212> DNA
   <213> Artificial
<220>
   <223> Artificially synthesized primer sequence
<400> 17
   ggtgaaggtc ggagtcaacg 20
<210> 18
   <211> 22
   <212> DNA
   <213> Artificial
<220>
   <223> Artificially synthesized primer sequence
<400> 18
   tgggtggaat catattggaa ca 22
<210> 19
   <211> 19
   <212> DNA
   <213> Artificial
<220>
   <223> Artificially synthesized primer sequence
<400> 19
   ttgccgacag gatgcagaa 19
<210> 20
   <211> 20
   <212> DNA
   <213> Artificial
<220>
   <223> Artificially synthesized primer sequence
<400> 20
   gccgatccac acggagtact 20

## Description

### [Technical Field]

The present invention relates to an artificial catalyst system substitutable for an *in vivo* acylation function. Particularly, the present invention relates to an artificial catalyst system substitutable for an acetylation function or a malonylation function.

### [Background Art]

I*n vivo* post-translational modifications of proteins play key roles in regulations of biological functions. Post-translational modifications include various reactions such as methylation and phosphorylation, and a typical example thereof includes histone acetylation. Histones are main proteins composing a chromosome, and have a role in storing DNA in the nucleus, the DNA being wound around the histones. Besides, histones are actively involved in dynamic regulations of chromosome structure and gene transcriptions following acetylation of lysine residues in histones with a histone acetyltransferase, and deacetylation thereof with a histone deacetylase (NPL 1) .

Enhancing histone acetylation leads to gene transcription enhancement. Accordingly, anticancer agents have been developed which inhibit histone deacetylases and thereby promote the transcription of a tumor suppressor gene. Atypical example thereof includes Zolinza, a drug against cutaneous T cell lymphoma (NPL 2) .

Many small molecule drugs, which are for treatment of diseases, exhibit their efficacy by controlling, particularly inhibiting, the functions of endogenous enzymes of the organisms. However, an approach using a small molecule drug with such actions has a problem that the therapeutic effect cannot be expected in a case where a disease is caused by a loss or inactivation of an enzyme itself. For example, an endogenous histone acetyltransferase is inactivated in some B-cell lymphomas. For this reason, it is concerned that acetylation enhancement cannot be expected from a treatment with a histone deacetylase inhibitor (NPL 3).

Furthermore, recently, malonylation and succinylation have been found out as the other acylation modifications on histones than acetylation. It has been suggested that these modifications play important roles in the structure and function of histones (NPL 4).

### [Citation List]

### [Non Patent Literatures]

[NPL 1] Brownell, J. E. et al., Cell, 1996, 84, 843-851
[NPL 2] Richon, V. M. et al., Proc. Natl. Acad. Sci. USA, 1996, 93, 5705-5708
[NPL 3] Pasqualucci, L. et al., Nature, 2011, 471, 189-195
[NPL 4] Xie, Z. et al., Mol Cell Proteomics, 2012 May; 11 (5), 100-107

### [Summary of Invention]

The invention provides an agent for acylating a chromosome protein, the agent comprising a combination of the following compound (a) and the following compound (b) :
(a) a compound having a chromosome-localizable activity and a nucleophilic catalytic activity, wherein the compound has a structure in which five or more molecules of 4-dimethylaminopyridine are linked with linkers and in which the carbon of a methyl group or position 3 or 5 of the pyridine in each 4-dimethylaminopyridine is linked to the linker; and
(b) a compound having a chromosome-localizable activity and a lysine acylating activity, wherein the compound has a structure in which an acylating agent and a cationic compound are linked, wherein said acylating agent is N-methoxydiacetamide or 4-malonyloxybenzamide and said cationic compound is a cationic polymer which is polyarginine.

The invention also provides a compound having a chromosome-localizable activity and a nucleophilic catalytic activity, wherein the compound has a structure in which five or more molecules of 4-dimethylaminopyridine are linked with linkers and in which the carbon of a methyl group or position 3 or 5 of the pyridine in each 4-dimethylaminopyridine is linked to the linker.

The invention further provides a compound having a chromosome-localizable activity and a lysine acylating activity, wherein the compound has a structure in which an acylating agent and a cationic compound are linked, wherein said acylating agent is N-methoxydiacetamide or 4-malonyloxybenzamide and said cationic compound is a cationic polymer which is polyarginine.

The invention additionally provides an agent for use in a method of treatment by inducing an expression of any one of p53, p21, and p27 in a cancer cell, the agent comprising a combination of a compound according to the invention and a compound according to the invention.

The invention also provides an agent for use in treating a disease caused by a reduction in chromosome protein acetylation, the agent comprising a combination of a compound according to the invention and a compound according to the invention.

### [Technical Problem]

Cells are a place where integrated chemical reactions take place, and life activities are supported by the activity of enzymes, one type of catalysts. Thus, if an artificial catalyst system substitutable for *in vivo* enzyme functions can be developed, the system will enable effective treatments against diseases caused by a loss or inactivation of an enzyme as described above. Accordingly, an object described herein is to develop an artificial catalyst system substitutable for an *in vivo* enzyme function, and to realize a medical treatment based on a novel concept of "catalysis medicine" in which the system is introduced into cells. As an example of this "catalysis medicine, " an object of the present disclosure is to provide an artificial catalyst system substitutable for an *in vivo* histone acylation function.

### [Solution to Problem]

In order to achieve the above objects, the present inventors first made efforts to artificially reconstruct an acetylation function among *in vivo* histone acylation functions. The inventors selected 4-dimethylaminopyridine (DMAP), widely used as a nucleophilic catalyst, as a representative of a catalyst, and selected N-methoxydiacetamide (NMD), known to acetylate a primary amine in water under mild conditions, as a representative of an acetylating agent. The use of DMAP is discussed in HANGXIANG WANG ET AL:JOURNAL OF THE AMERICAN CHEMICAL SOCIETY, vol. 133, no. 31, 10, pages 12220-12228; YOICHIRO KOSHI ET AL:JOURNAL OF THE AMERICAN CHEMICAL SOCIETY, vol. 130, no. 1, pages 245-251; and TAKAHIRO HAYASHI ET AL:JOURNAL OF THE AMERICAN CHEMICAL SOCIETY, vol. 135, no. 33 pages 12252-12258; and alternative acylating agents are disclosed in SHO-HEI FUJISHIMA ET AL: JOURNAL OF THE AMERICAN CHEMICAL SOCIETY, vol. 134, no. 9,pages 3961-3964. Then, cells were treated with a combination of these molecules to investigate whether or not the combination enhanced acetylation of chromosome proteins including histones. Although the combination of these molecules highly acetylated proteins present in the cytoplasm, the acetylation of chromosome proteins including histones was not, however, enhanced. In addition, the investigation on the intracellular localization of these molecules demonstrated no accumulation in the nucleus and the chromosomes, in consistent with the above result.

Next, the present inventors made efforts for improvement such that these molecules had accessibility to the vicinity of chromosomes in a cell. To be more specific, the inventors focused on the fact that DNA in a chromosome is a polyanion, and investigated whether or not it was possible to improve the accessibility to chromosomes through electrostatic interaction by imparting a polycationic nature to the molecules . In this respect, since a basic catalyst 4-dimethylaminopyridine (DMAP) exhibits a cationic nature by itself in water, the inventors prepared multiple molecules of DMAP which were linked to each other with a linker to enhance the nature as a cation. Then, the prepared molecules were introduced into cells to investigate the localization. As a result, it was revealed that when three molecules of 4-dimethylaminopyridine were linked, the molecules exhibited partial chromosome localization; when five or more molecules thereof were linked, the molecules exhibited significant chromosome localization.

On the other hand, since an acetylating agent N-methoxydiacetamide (NMD) does not exhibit a cationic nature by itself in water, another molecule exhibiting a cationic nature was linked thereto. A cationic polymer polyarginine has been known as a cell membrane-permeable peptide, but the localization to chromosomes has not been known. Hence, a polyarginine was linked to N-methoxydiacetamide with a linker to investigate the intracellular localization. As a result, it was revealed that the molecule thus synthesized exhibited the localization to chromosomes.

Since both of the "chromosome-localizable nucleophilic catalyst" and the "chromosome-localizable acetylating agent" were successfully prepared, the present inventors then investigated the ability to acetylate chromosome proteins. The result revealed that the treatment with the combination of these molecules enhanced the acetylation specific to chromosome proteins including histones, independently of an endogenous histone acetyltransferase and regardless of the cell type (this acetylation will be referred to as "synthetic chromosome acetylation"). The acetylation of lysine present in a globular region of histone H3 was particularly significantly enhanced among histones.

Further, the influence on the cell growth by the synthetic chromosome acetylation with a combination of these molecules was analyzed. As a result, surprisingly, it was revealed that the synthetic chromosome acetylation did not influence the cell cycle of normal cells but promoted the cell cycle arrest specific to cancer cells. This cancer-cell specific cell cycle arrest was dependent on a tumor suppressor gene p53, and the expressions of p53 and cyclin-dependent kinase inhibiting factorsp21 and p27 were induced in cancer cells.

Thus, the present inventors successfully established an artificial catalyst system substitutable for an *in vivo* histone acetylation function, by such an approach of synthetic chromosome acetylation using a combination of a chromosome-localizable nucleophilic catalyst and a chromosome-localizable acetylating agent.

After this success, the present inventors further made efforts to establish an artificial catalyst system substitutable for a histone malonylation function. To be more specific, the inventors selected 4-malonyloxybenzamide as a representative of a malonylating agent, and linked a polyarginine thereto with a linker as in the above case of the acetylating agent to thereby prepare a "chromosome-localizable malonylating agent." Then, the chromosome-localizable malonylating agent and the aforementioned chromosome-localizable nucleophilic catalyst were combined to investigate the ability to malonylate chromosome proteins. The result revealed that the treatment with a combination of these molecules significantly malonylated lysines of histones H3 and H4 (this malonylation will be referred to as "synthetic chromosome malonylation").

From the foregoing, the present inventors have found out that "synthetic chromosome acylation" using a combination of a chromosome-localizable nucleophilic catalyst and a chromosome-localizable acylating agent is substitutable for an *in vivo* acylation function. In addition, the inventors have found out that the utilization of this artificial catalyst system enables treatments against diseases caused by a reduction in an *in vivo* acylation function, and so forth. These discoveries have led to the completion of the present invention.

The present invention relates to an artificial catalyst system for specifically acylating a chromosome protein, molecules used in the artificial catalyst system, and applications of the artificial catalyst system to medical treatments and so forth, as defined in the claims.

### [Advantageous Effects of Invention]

Described herein is an artificial catalyst system by such an approach of synthetic chromosome acylation using a combination of a chromosome-localizable nucleophilic catalyst and a chromosome-localizable acylating agent.

The acetylation induced by the artificial catalyst described herein is independent of an *in vivo* histone acetyltransferase. The main function of a histone acetyltransferase exhibiting its function through an interaction with another protein *in vivo,* and so forth, has been successfully substituted with the artificial catalyst system by which the nucleophilic catalyst and the acetylating agent are migrated to a chromosome for the synthetic chromosome acetylation.

The artificial catalyst system described herein makes it possible to, for example, effectively treat diseases caused by a reduction in chromosome protein acetylation. The acetylation is enhanced by this artificial catalyst system regardless of the cell type, but the actions thereof on cell cycle arrest and so forth are specific to cancer cells. Moreover, the site where the acetylation is enhanced is different from a site where acetylation is enhanced by an action of a conventional histone deacetylase inhibitor. Thus, novel and specific pharmacological actions can also be expected from medical treatments using the artificial catalyst system described herein.

The artificial catalyst system described herein makes it possible to induce not only histone acetylation but also malonylation. Thus, it is conceivable that acylations including acetylation and malonylation can be carried out widely on histones on the basis of the basic principle of this system.

### [Brief Description of Drawings]

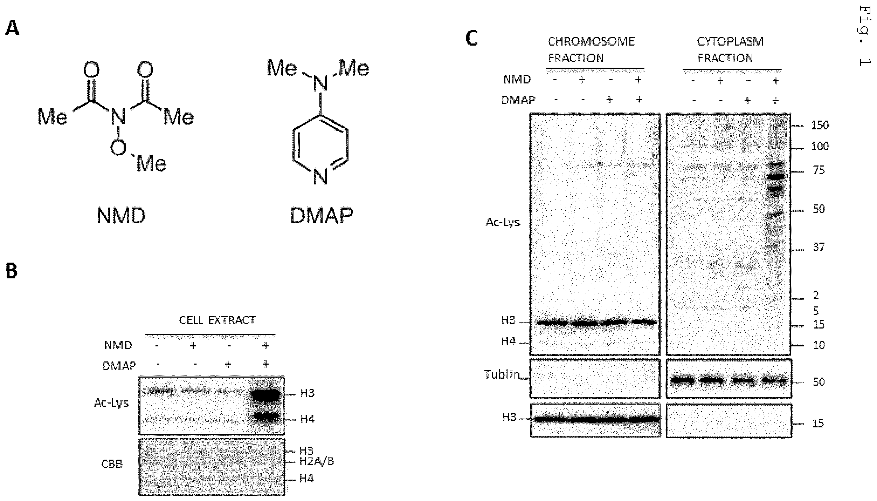

[Fig. 1] Fig. 1 is a figure for illustrating the structures of 4-dimethylaminopyridine (DMAP) and N-methoxydiacetamide (NMD), as well as acetylation enhancement with a combination of these. A shows drawings for illustrating the structures of DMAP and NMD, B shows photographs for illustrating the acetylation in cell extracts, and C shows photographs for illustrating the acetylation in human MCF7 cells.

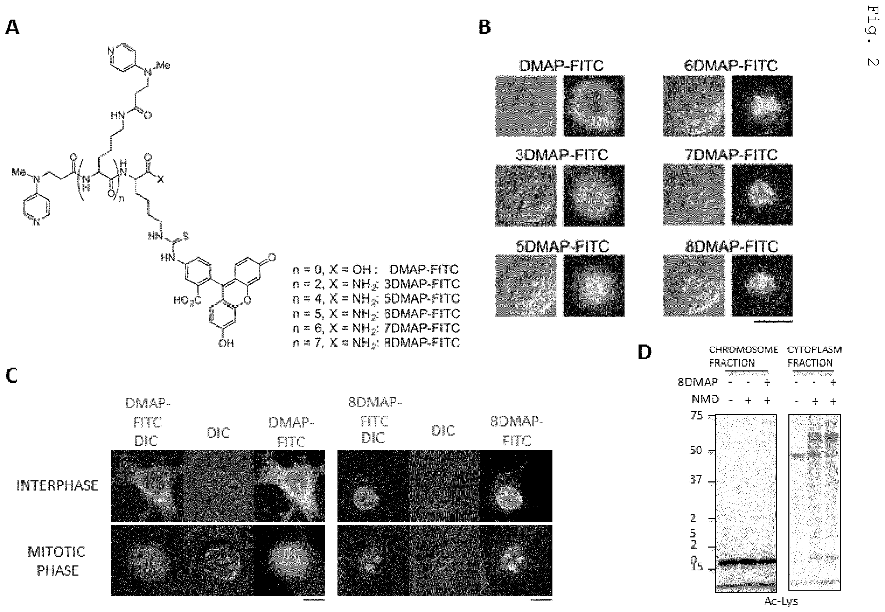

[Fig. 2] Fig. 2 is a figure for illustrating the characteristics of chromosome-localizable nucleophilic catalysts. A shows drawings for illustrating the structure of XDMAP-FITC, B shows photographs for illustrating the localization in human Hela cells, C shows photographs for illustrating the localization in human MCF7 cells, and D shows photographs for illustrating the acetylation in the human Hela cells.

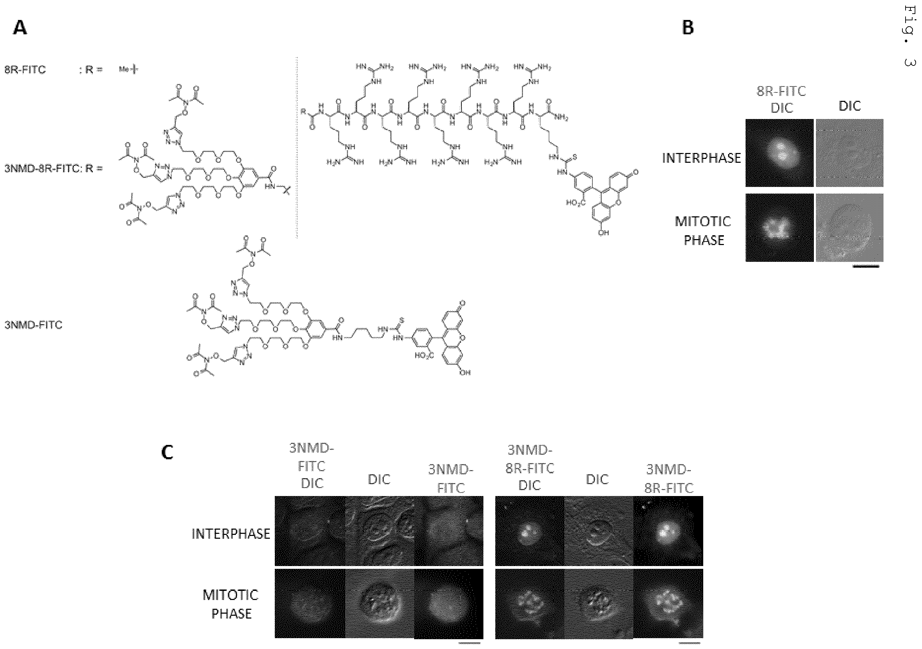

[Fig. 3] Fig. 3 is a figure for illustrating the characteristics of chromosome-localizable acetylating agents. A shows drawings for illustrating the structures of 8R-FITC, 3NMD-FITC, and 3NMD-8R-FITC, B shows photographs for illustrating the localization of 8R-FITC in human Hela cells, and C shows photographs for illustrating the localization in human MCF7 cells.

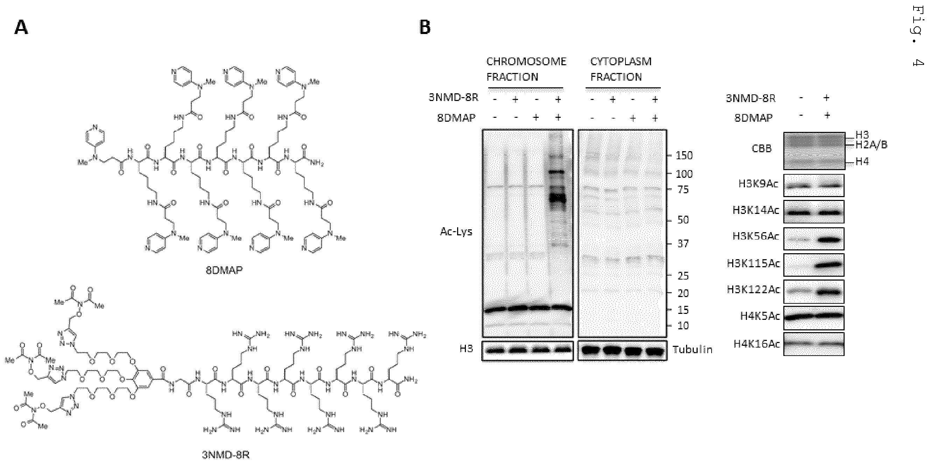

[Fig. 4] Fig. 4 is a figure for illustrating the chromosome protein-selective acetylation enhancement by an artificial catalyst system described herein (a combination of the chromosome-localizable nucleophilic catalyst and the chromosome-localizable acetylating agent). A shows drawings for illustrating the structures of 8DMAP and 3NMD-8R, and B shows photographs for illustrating the chromosome-selective acetylation in human MCF7 cells.

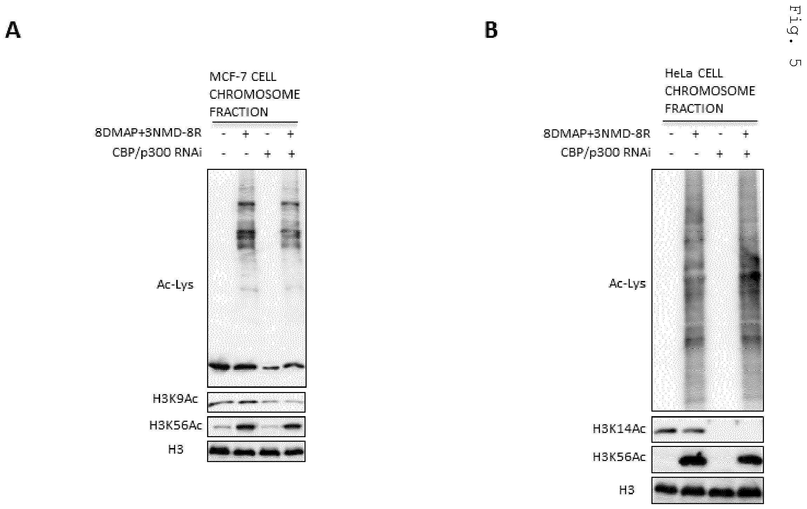

[Fig. 5] Fig. 5 is a figure for illustrating the endogenous-HAT independence of the acetylation with the artificial catalyst system described herein. A shows photographs for illustrating the result of HAT knockdown in human MCF7 cells, and B shows photographs for illustrating the result of the HAT knockdown in human Hela cells.

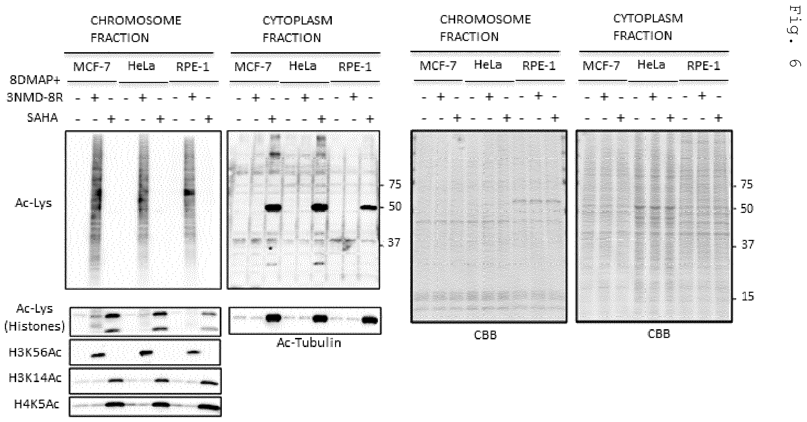

[Fig. 6] Fig. 6 is a figure for illustrating the non-cell-specificity of the acetylation with the artificial catalyst system described herein. The comparisons among human MCF7 cells, human Hela cells, and human RPE-1 cells are shown. Moreover, the comparisons with the action of a histone deacetylase inhibitor SAHA are also shown together.

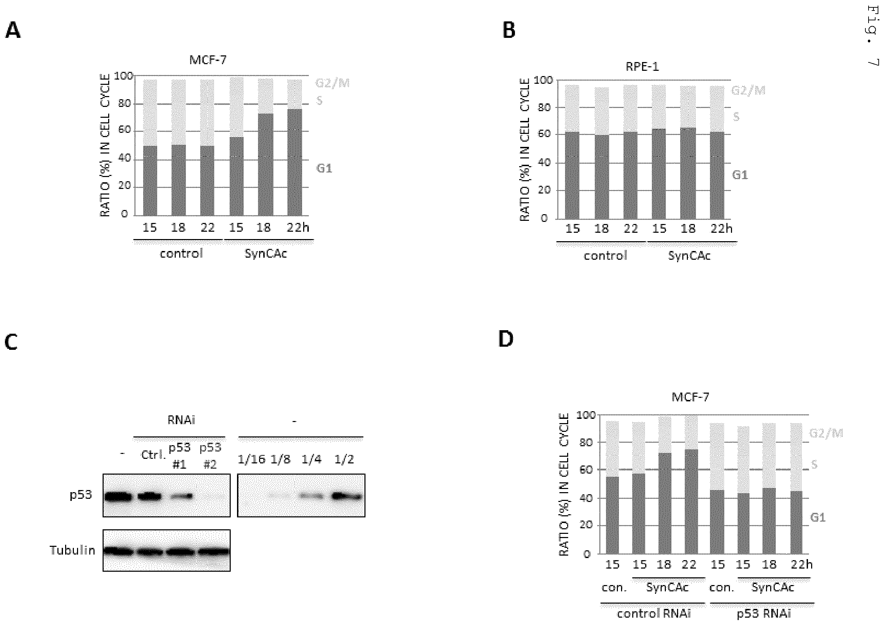

[Fig. 7] Fig. 7 is a figure for illustrating the influence on the cell cycle by the acetylation enhancement with the artificial catalyst system described herein. A is a graph for illustrating the result of the cell cycle analysis in human MCF7 cells, B is a graph for illustrating the result of the cell cycle analysis in human RPE-1 cells, C shows photographs for illustrating the evaluation of p53 knockdown, and D is a graph for illustrating the result of the cell cycle analysis with the p53 knockdown.

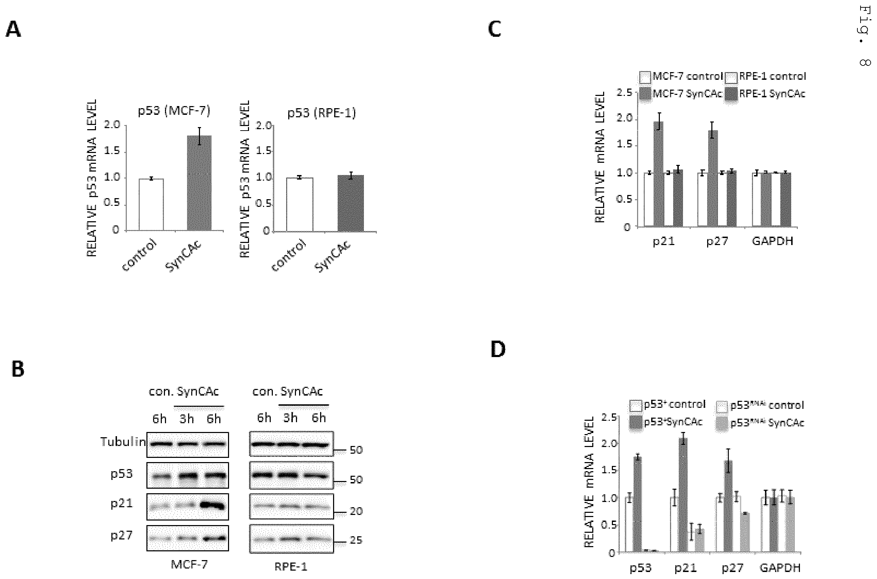

[Fig. 8] Fig. 8 is a figure for illustrating the influence on the expressions of p53 and genes related thereto in the cancer cell line by the acetylation with the artificial catalyst system described herein. A shows graphs for illustrating the analysis result of p53 at an mRNA level, B shows photographs for illustrating the analysis result of p53, p21, and p27 at a protein level, C is a graph for illustrating the analysis result of p21 and p27 at an mRNA level, and D is a graph for illustrating the analysis result of p21 and p27 at an mRNA level with the p53 knockdown.

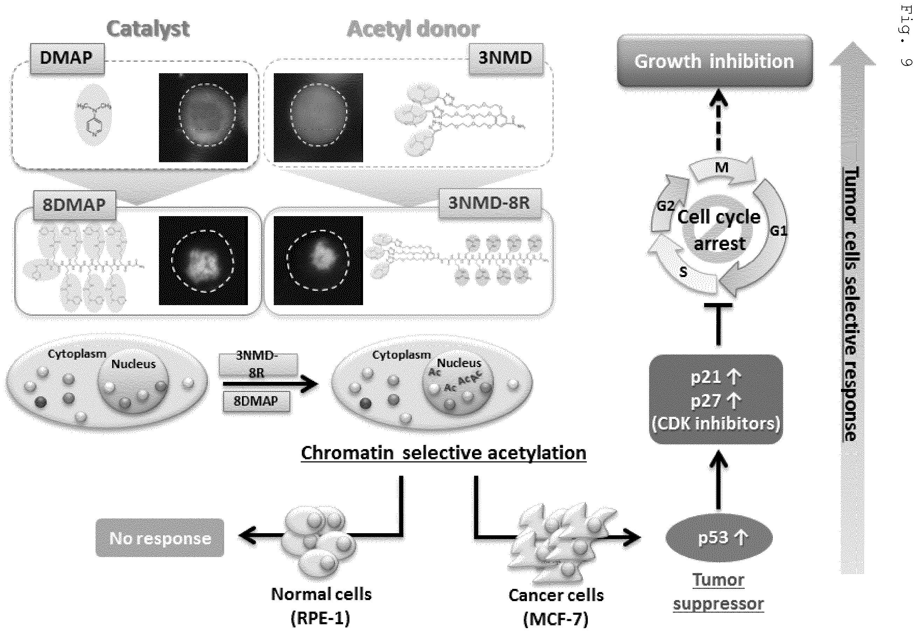

[Fig. 9] Fig. 9 is a figure for summarizing the artificial catalyst system described herein (acetylation).

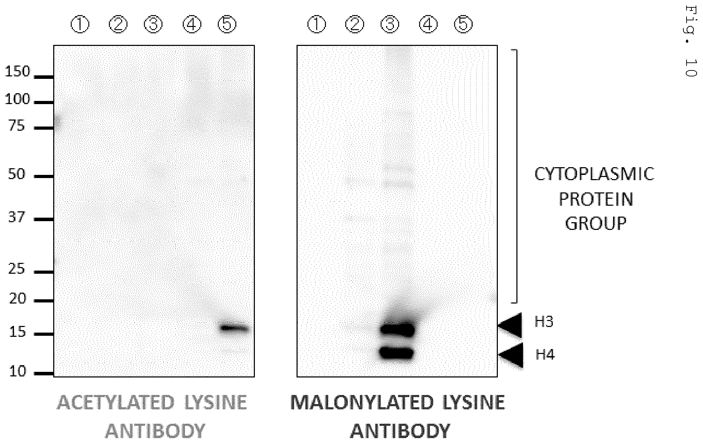

[Fig. 10] Fig. 10 is a figure for illustrating the chromosome protein-selective acylation with the artificial catalyst system described herein (a combination of the chromosome-localizable nucleophilic catalyst and a chromosome-localizable malonylating agent, or the combination of the chromosome-localizable nucleophilic catalyst and the chromosome-localizable acetylating agent). The photograph on the right of the figure illustrates the result of detecting the malonylation, and the photograph on the left of the figure illustrates the result of detecting the acetylation. The explanation of each lane is as follows. 1: no compound treatment, 2: only 3Mal-BA-8R was added, 3: L-8DMAP and 3Mal-BA-8R were added, 4: only 3NMD-8R was added, and 5: L-8DMAP and 3NMD-8R were added.

### [Description of Embodiments]

### <Chromosome-Localizable Nucleophilic Catalyst>

The present invention relates to a chromosome-localizable nucleophilic catalyst, that is, a compound having a chromosome-localizable activity and a nucleophilic catalytic activity. The chromosome-localizable nucleophilic catalyst is one constituent of an artificial catalyst system described herein, and is capable of enhancing chromosome protein acylation when introduced to cells in combination with a chromosome-localizable acylating agent to be described later.

Herein, the "chromosome-localizable activity" and related terms mean such a nature that when the compound is introduced into cells, the compound is located more in chromosomes than in the other regions (for example, cytoplasm). Whether or not a compound has a chromosome-localizable activity can be evaluated by: conjugating a fluorescent dye such as fluorescein isothiocyanate (FITC) to the compound, introducing the conjugate into cells, and observing the cells with a fluorescence microscope. Moreover, the term "nucleophilic catalyst" means a reaction catalyst capable of expressing its catalytic action through a nucleophilic attack, and the term "nucleophilic catalytic activity" means a catalytic action of the catalyst through a nucleophilic attack.

The present Examples have revealed that imparting a polycationic nature to a nucleophilic catalyst makes it possible to impart a chromosome-localizable activity to the nucleophilic catalyst. The chromosome-localizable nucleophilic catalyst described herein typically has a structure in which an effective number of cationic nucleophilic catalysts for the chromosome localization are linked. Nevertheless, the essence of the activity of the chromosome-localizable nucleophilic catalyst described herein is the chromosome-localizable activity and the nucleophilic catalytic activity. Hence, as long as the two activities are retained, the chromosome-localizable nucleophilic catalyst may have a structure in which the cationic nucleophilic catalysts are linked to another cationic compound, or may have a structure in which a nucleophilic catalyst not exhibiting a cationic nature is linked to another cationic compound. The linking may be a linkage with a linker.

Examples of the "cationic nucleophilic catalyst" include pyridine-based catalysts such as 4-dimethylaminopyridine (DMAP), imidazole-based catalysts such as 1-methylimidazole, bicyclooctane-based catalysts such as 1,4-diazabicyclo[2.2.2]octane, and phosphine-based catalysts such as tributylphosphine, but are not limited thereto. The present Examples have revealed that in the case of using 4-dimethylaminopyridine as the cationic nucleophilic catalyst, when five or more molecules thereof are linked, the molecules exhibit significant chromosome localization. Thus, the chromosome-localizable nucleophilic catalyst described herein is preferably a compound having a structure in which five or more molecules of 4-dimethylaminopyridine are linked. More preferable is a compound having a structure in which six or more molecules of 4-dimethylaminopyridine are linked, furthermore preferable is a compound having a structure in which seven or more molecules of 4-dimethylaminopyridine are linked, and still furthermore preferable is a compound having a structure in which eight or more molecules of 4-dimethylaminopyridine are linked.

In the case where multiple molecules of 4-dimethylaminopyridine are linked with a linker, the chromosome-localizable nucleophilic catalyst described herein preferably has a structure in which carbon of a methyl group or position 3 or 5 of pyridine in 4-dimethylaminopyridine is linked to the linker, and particularly preferably has a structure in which the carbon of a methyl group is linked to the linker, from the viewpoints of maintaining the nucleophilic catalytic activity, and so forth.

Although a preferable linker is exemplified in the present Examples, the linker is not particularly limited, as long as the chromosome-localizable activity and the nucleophilic catalytic activity of a compound to be synthesized are not inhibited. Thus, those skilled in the art can select or synthesize other linkers as appropriate and utilize them accordingly. Examples of the other utilizable linkers include cyclic peptide linkers, polyamideamine linkers, N-methyl peptide linkers, peptoid linkers, and ROMP polymer linkers. Numerous structures of the other linkers are conceivable, and several typical examples of the structure are shown below. 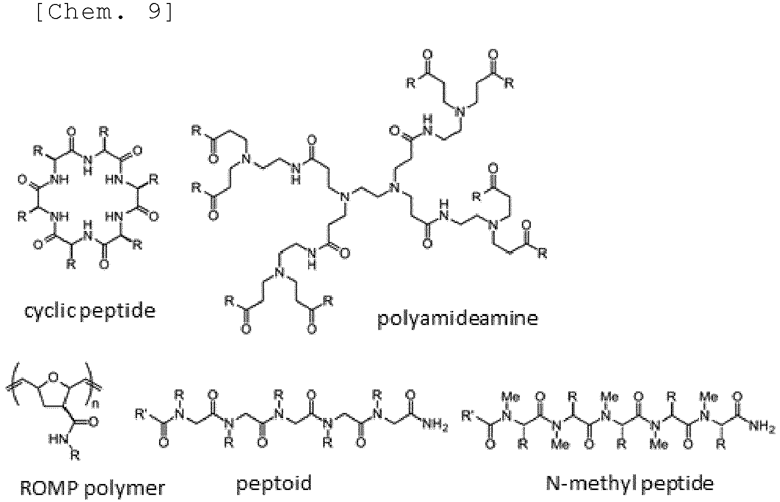 (each R and R' represents any functional group which may be the same or different from each other. n represents the number of repetitions of the structure.)

The cationic nucleophilic catalyst may be linked, for example, directly to R or R' of any of the above-described linker structures, or alternatively indirectly thereto with another linker structure. As long as a compound to be synthesized finally has the chromosome-localizable activity and the nucleophilic catalytic activity, the cationic nucleophilic catalyst does not necessarily have to be linked to all of Rs and R'. Moreover, the repetitions in the structure of the ROMP polymer linker should be determined within such a range that the finally synthesized compound has the chromosome-localizable activity and the nucleophilic catalytic activity.

As long as the chromosome-localizable nucleophilic catalyst described herein has, as a whole, a polycationic nature equivalent to or greater than that exhibited by the five molecules of 4-dimethylaminopyridine, part or all of the polycationic nature may be relied on another cationic compound. Examples of the another cationic compound include compounds having an amino group, a monoalkylamino group, a dialkylamino group, an imino group, a guanidino group, or the like. Examples of such compounds include basic amino acids such as arginine, monomethyllysine, and dimethyllysine, modified products thereof, homopolymers thereof (such as polyarginine, poly(monomethyllysine), poly(dimethyllysine)) and copolymers thereof, polyethylenimine, and DNA-binding dyes. Linking these cationic compounds makes it possible to impart a chromosome-localizable activity to a nucleophilic catalyst not exhibiting a cationic nature.

As the chromosome-localizable nucleophilic catalyst, the following compound having a linear structure can be suitably used.

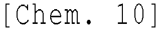

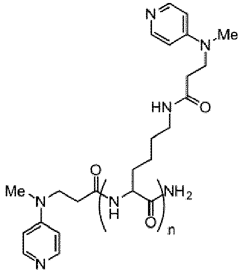

Here, n represents a natural number within such a range that the compound has the chromosome-localizable activity and the nucleophilic catalytic activity. Normally, n is within a range of 4 to 14, preferably 6 to 9.

In the present Examples, the following compound having a linear structure is used as an example.

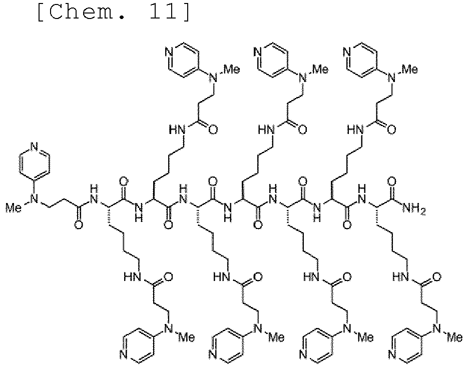

As the chromosome-localizable nucleophilic catalyst, the following compound having a cyclic structure can also be suitably used.

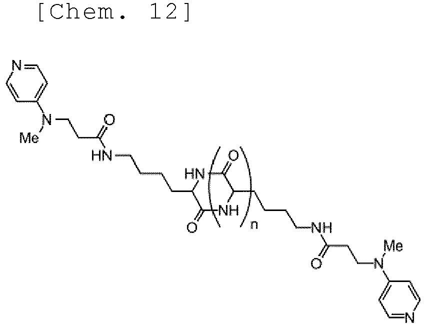

Here, n represents a natural number within such a range that the compound has the chromosome-localizable activity and the nucleophilic catalytic activity. Normally, n is within a range of 4 to 14, preferably 6 to 9.

In the present Examples, the following compound having a cyclic structure is used as an example.

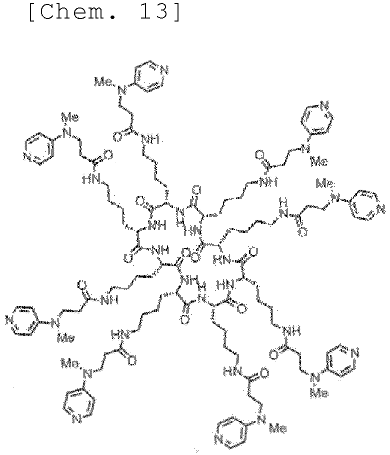

Note that although polylysine is used as a linker in the above compounds, an ε-amino group at a side chain of this polylysine is modified. Thus, it should be noted that the polylysine structure itself no longer exhibits a cationic nature in these compounds.

### <Chromosome-Localizable Acylating Agent>

The present invention relates to a chromosome-localizable acylating agent, that is, a compound having a chromosome-localizable activity and a lysine acylating activity. The chromosome-localizable acylating agent is another constituent of the artificial catalyst system described herein, and is capable of enhancing chromosome protein acylation when introduced to cells in combination with the above-described chromosome-localizable nucleophilic catalyst.

The present Examples have revealed that imparting a polycationic nature to an acylating agent makes it possible to impart a chromosome-localizable activity to the acylating agent. Thus, the chromosome-localizable acylating agent described herein has a structure in which an acylating agent and a cationic compound are linked. The linking may be a linkage with a linker.

The "cationic compound" is not particularly limited, except by the scope of the claim, as long as it is capable of imparting a chromosome-localizable activity to the acylating agent. Examples of cationic compounds include compounds having an amino group, a monoalkylamino group, a dialkylamino group, an imino group, a guanidino group, or the like. The cationic compound is preferably a cationic polymer, and examples thereof include basic amino acids such as arginine, monomethyllysine, and dimethyllysine, homopolymers of modified products of basic amino acids (such as polyarginine, poly(monomethyllysine), poly(dimethyllysine)) and copolymers thereof, polyethylenimine, and DNA-binding dyes. In the compounds of the invention, the cationic compound is polyarginine.

The acylating agent of the invention is defined in the claims. Examples of the "acylating agent" described herein include acetylating agents and malonylating agents, and also include other acylating agents than these. The acylating agent can be selected as appropriate, depending on the type of the targeted acylation.

Examples of the "acetylating agent" include N-methoxydiacetamide, phenyl thioacetate, and 4-nitrophenyl acetate, but are not limited thereto. In preparing a chromosome-localizable acetylating agent, this agent may contain several structures of these acetylating agents.

In the case where N-methoxydiacetamide is linked to the cationic compound with a linker, the resultant preferably has a structure in which carbon of a methoxy group in N-methoxydiacetamide is linked to the linker, from the viewpoints of maintaining the acetylating activity, and so forth.

Examples of the "malonylating agent" include 4-malonyloxybenzamide, phenyl thiomalonate, and 4-nitrophenyl malonate, but are not limited thereto. In preparing a chromosome-localizable malonylating agent, this agent may contain several structures of these malonylating agents.

In the case where 4-malonyloxybenzamide is linked to the cationic compound with a linker, the resultant preferably has a structure in which an amide group in 4-malonyloxybenzamide is linked to the linker, from the viewpoints of maintaining the malonylating activity, and so forth.

Although a preferable linker is exemplified in the present Examples, the linker is not particularly limited, as long as the chromosome-localizable activity and the lysine acylating activity of a compound to be synthesized are not inhibited. Thus, those skilled in the art can select or synthesize other linkers as appropriate and utilize them accordingly. Examples of the other utilizable linkers include alkyl linkers, oligoethylene glycol linkers, and peptide linkers. Numerous structures of the other linkers are conceivable, and several typical examples of the structure are shown below. 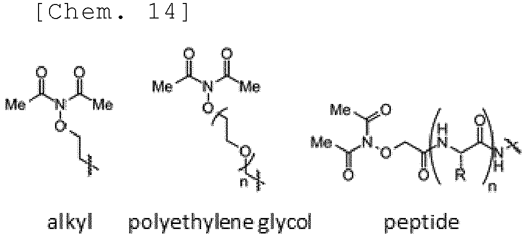 (R represents any functional group, and n represents the number of repetitions of the structure.)

In these examples, one end of each linker is shown to have a structure in which N-methoxydiacetamide is linked as an acetylating agent, but other acylating agents may be linked. The cationic compound may be linked, for example, directly to an end of two ends of a linker, the end being opposite to an end where an acylating agent is linked, or alternatively indirectly thereto with another linker structure. In a case where an amino acid having a repeated structure in the peptide linker is a basic amino acid and the repeated structure can guarantee the chromosome-localizable activity, it is not necessary to link another cationic compound to the end opposite to the end where the acylating agent is linked.

As the chromosome-localizable acylating agent, an acetylating agent or a malonylating agent having the following structures can be suitably used.

### [Acetylating Agent]

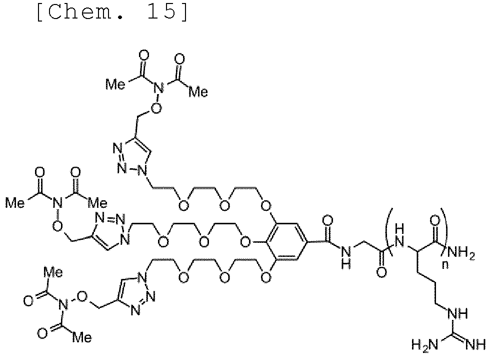

Here, n represents a natural number within such a range that the compound has the chromosome-localizable activity and a lysine acetylating activity. Normally, n is within a range of 5 to 15, preferably 5 to 10.

In the present Examples, a compound having the following structure is used as an example of the acetylating agent.

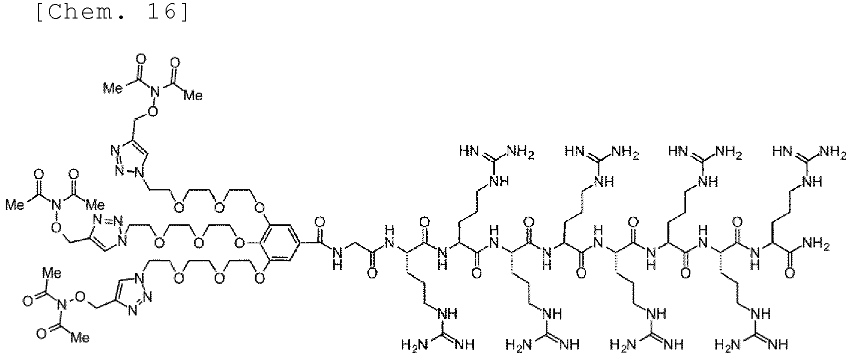

### [Malonylating Agent]

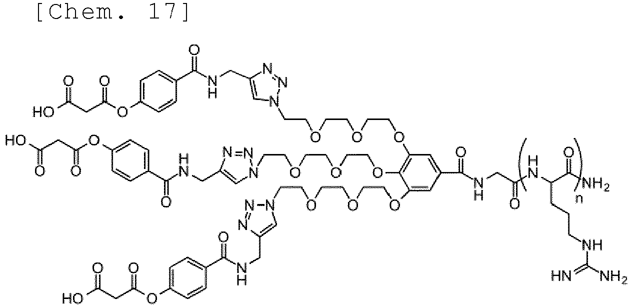

Here, n represents a natural number within such a range that the compound has the chromosome-localizable activity and a lysine malonylating activity. Normally, n is within a range of 5 to 15, preferably 5 to 10.

In the present Examples, a compound having the following structure is used as an example of the malonylating agent.

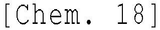

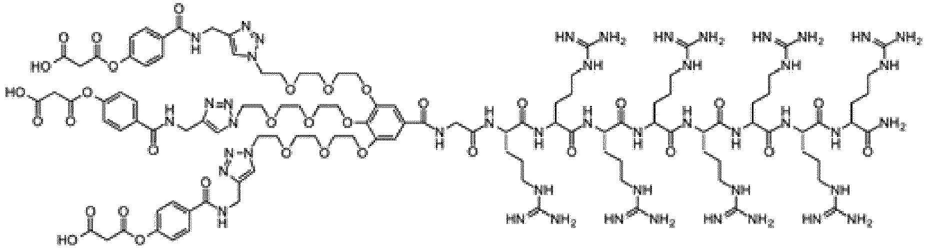

### <Combination Agents>

### -Chromosome-Protein Acylating Agent-

The present Examples have revealed that it is possible to enhance chromosome protein acylation when a combination of the chromosome-localizable nucleophilic catalyst and the chromosome-localizable acylating agent is introduced to cells . Thus, an agent for acylating a chromosome protein is described herein, the agent comprising a combination of the above-described chromosome-localizable nucleophilic catalyst and the above-described chromosome-localizable acylating agent. Also described herein is a method for acylating a chromosome protein by using the combination. The "chromosome protein" to be acylated is mainly a histone (particularly, histones H3 and H4).

In carrying out the acetylation, the acetylation of lysine (K56, K115, K122) present in a globular region of histone H3 is particularly enhanced. The region where the acetylation is enhanced is different from a case of a treatment with a known histone deacetylase inhibitor (see Fig. 6) . Hence, the chromosome-protein acetylating agent makes it possible to exert a novel action different from that of a known histone deacetylase inhibitor. Further, it is also possible to enhance acetylation of chromosome proteins other than the histone (see Fig. 4B).

The acylation of lysine residues of chromosome proteins can be evaluated, for example, by a western blotting method using an anti-acetylated lysine antibody or an anti-malonylated lysine antibody. In a case where the acylation of a particular lysine residue (for example, K56, K115, or K122 of histone H3) is to be evaluated, an antibody specific to the acylated particular lysine residue should be used.

### -Expression Inducer for p53 and the like-

Furthermore, the present Examples have revealed that the chromosome protein acetylation induces the expressions of a tumor suppressor gene p53 and cyclin-dependent kinase inhibiting factors p21 and p27 in cancer cells. Thus, an agent for inducing an expression of any one of p53, p21, and p27 in a cancer cell may comprise a combination of the above-described chromosome-localizable nucleophilic catalyst and the above-described chromosome-localizable acetylating agent. Also described herein is a method for inducing an expression of any one of p53, p21, and p27 in a cancer cell by using the combination.

Here, the term "expression" means to include both an expression at an mRNA level (transcription) and an expression at a protein level (translation). "Inducing" an expression and related terms mean that the expression level is increased more than before the combination is allowed to exhibit the action.

The expression at an mRNA level can be evaluated, for example, by a real-time PCR method using a specific primer, and the expression at a protein level can be evaluated, for example, by a western blotting method using a specific antibody.

Note that the base sequence of a cDNA of typical human-derived p53 (gene name: TP53) and the amino acid sequence of a protein thereof are listed under the registration number CAA26306 in the ENA (European Nucleotide Archive) database; the base sequence of a cDNA of typical human-derived p21/CIP1 (gene name: CDKN1A) and the amino acid sequence of a protein thereof are listed under the registration number AAA16109 in the ENA database; the base sequence of a cDNA of typical human-derived p27/KIP1 (gene name: CDKN1B) and the amino acid sequence of a protein thereof are listed under the registration number AAL78041 in the ENA database. It should be understood that these sequences are merely examples, and the sequences may differ among individuals and may mutated in nature.

### -Therapeutic Agent for Disease Caused by Reduction in Chromosome Protein Acetylation-

Also described herein is an agent for treating a disease caused by a reduction in chromosome protein acetylation, the agent comprising a combination of the above-described chromosome-localizable nucleophilic catalyst and the above-described chromosome-localizable acetylating agent. In addition, a method for treating a disease caused by a reduction in chromosome protein acetylation may comprise administering the combination to a patient.

The present Examples have revealed that the combination has an action of specifically arresting the cell cycle of cancer cells. Thus, the "disease caused by a reduction in chromosome protein acetylation" is preferably a cancer. Since an endogenous histone acetyltransferase is inactivated in some B-cell lymphomas, it is concerned that acetylation enhancement cannot be expected from a treatment with a histone deacetylase inhibitor (Pasqualucci, L. et al., Nature 2011, 471, 189-195). Even if an endogenous histone acetyl transferase is inactivated, the therapeutic agent described herein is able to complement the function and can exhibit efficacy that cannot be achieved by conventional histone deacetylase inhibitors.

Moreover, the therapeutic agent described herein may be used in combination with other anticancer agents and other cancer treatment methods (for example, radiation therapy, immunotherapy). It has been suggested that histone acetylation decondenses the chromatin structure, thereby increases the DNA accessibility and enhances the sensitivity of chromosome DNA to a DNA-damaging agent and radiation (Oleinick et al., Int. J. Radiat. Biol. 1994, 66, 523-529, Gorisch SM, et al., J Cell Sci 2005, 118, 5825-5834, Camphausen K, et al. Int J Cancer 2005, 114, 380-366, Karagiannis TC & El-Osta A. Oncogene 2006, 25, 3885-3893, Kim MS, et al., Exp Cell Res 2005, 306, 94-102, Kim MS, et al., Cancer Res 2003, 63, 7291-7300, Piacentini P, et al., Virchows Arch 2006, 448, 797-804). Since the therapeutic agent described herein is believed to have an activity of acetylating chromosome proteins and decondense the chromatin structure, synergistic effects can be expected from the combination with a DNA targeting agent (for example, an anticancer agent having a DNA-damaging action) or a DNA-targeting treatment method (for example, radiation therapy).

In the case where the chromosome-localizable nucleophilic catalyst and the chromosome-localizable acetylating agent are used as a therapeutic agent, these compounds may be linked with a complement for enhancing therapeutically useful characteristics. Examples of typical useful characteristics thereof include: promoting the delivery of the compounds to a target region (for example, tumor), keeping the therapeutic window of the compounds in a target region, modifying the pharmacokinetic characteristics and pharmacodynamic characteristics of the compounds, and improving the therapeutic index or safety profile of the compounds. Moreover, as a preferable complement, it is possible to utilize, for example, an antibody capable of specifically recognizing a target region, and a ligand for a receptor expressed in a target region.

### -Formulation and Administration Mode-

In a case where the chromosome-protein acylating agent described herein is used as a drug, the chromosome-localizable nucleophilic catalyst and the chromosome-localizable acylating agent can be formulated as active ingredients by known pharmaceutical methods. The phrase that the agent "comprises a combination" of the chromosome-localizable nucleophilic catalyst and the chromosome-localizable acylating agent means that the agent may be in the form of a single agent comprising both of the chromosome-localizable nucleophilic catalyst and the chromosome-localizable acylating agent as active ingredients, or may be in the form of concomitant agents of a preparation comprising the chromosome-localizable nucleophilic catalyst as an active ingredient and a preparation comprising the chromosome-localizable acylating agent as an active ingredient.

Examples of a pharmacologically acceptable carrier used in the formulation include sterile water, physiological saline, vegetable oils, solvents, bases, emulsifiers, suspensions, surfactants, stabilizers, flavors, aromatic substances, excipients, vehicles, antiseptics, binders, diluents, isotonic agents, soothing agents, bulking agents, disintegrators, buffers, coating agents, lubricants, colorants, sweeteners, viscous agents, flavor modifiers, solubilizers, other additives, and the like, but are not limited thereto. The active ingredients can be made in various forms such as tablets, powders, granules, capsules, and liquids, in accordance with the purpose of the treatment and so forth. Moreover, the active ingredients can also be administered in the form of liposome delivery system. To the liposome, the aforementioned complements (such as, for example, antibody and ligand) can also be added to enhance therapeutically useful characteristics.

The administration to a patient can be carried out by either oral administration or parenteral administration. Examples of the parenteral administration include intravenous administration, intraarterial administration, intramuscular administration, intrathoracic administration, intraperitoneal administration, direct administration to a target site (for example, tumor), and the like. In the case where the agent described herein is concomitant agents, the preparations may be administered at the same time, or may be administered at different timings such that the combination effect will not be reduced.

The dose is not particularly limited, as long as the amount is effective for treating a target disease, and should be selected as appropriate in accordance with the age, weight, symptom, and health state of a patient, the progression of the disease, and so forth. How often the agent is administered is not particularly limited, either, and can be selected as appropriate in accordance with the purpose. For example, as the dose administered in a day, the agent may be administered once a day, or may be administered separately multiple times a day. When the agent described herein is administered to human, the range of the dose of the active ingredients per day is normally approximately 0.01 mg/kg body weight to approximately 500 mg/kg body weight, preferably approximately 0.1 mg/kg body weight to approximately 100 mg/kg body weight. When administered to a human, the agent described herein is administered preferably once a day, or preferably separately two to four times a day such that the administration is repeated at appropriate intervals.

Note that in a case where the chromosome protein acylating agent described herein or the expression inducer for p53 and the like is used as a reagent, the reagent may comprise, as necessary, other ingredients acceptable as a reagent such as sterile water, a physiological saline, a buffer, and a preservative, in addition to the active ingredients. The reagent can be administered to a target (such as, for example, cells, fractions thereof, tissues, experimental animals) in accordance with the purpose to thereby acylate a chromosome protein or induce an expression of p53 or the like.

### [Examples]

Hereinafter, the present invention will be described more specifically on the basis of Synthesis Examples and Examples. However, the present invention is not limited thereto.

### (Synthesis Example 1) Synthesis of Chromosome-Localizable Acylating Agent

### (1) Synthesis of Chromosome-Localizable Acetylating Agent

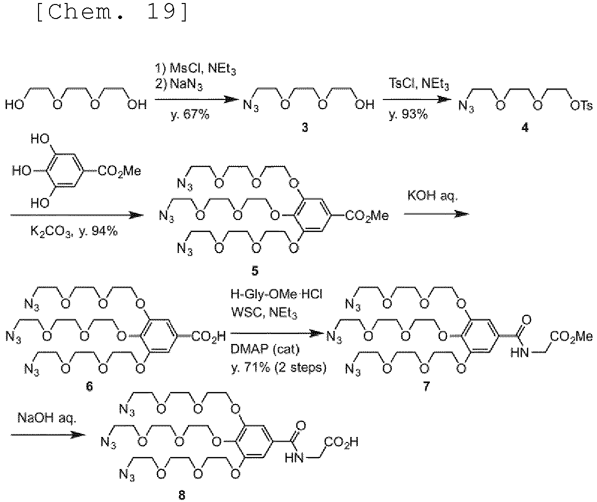

### (i) 2-(2-(2-Azidoethoxy)ethoxy)ethan-1-ol (Compound 3)

Triethylene glycol (10.0 g, 66.6mmol) was dissolved intetrahydrofuran (THF: 47.6ml), and triethylamine (NEt₃: 7.12 ml, 51.3 mmol) was added thereto at room temperature. Then, the reaction solution was cooled on ice. Methanesulfonyl chloride (MsCl: 1.84 ml, 23.8 mmol) was slowly added dropwise thereto. Subsequently, the resultant was stirred at room temperature for 9 hours . The reaction solution was concentrated, and thereafter the residue was dissolved in ethanol (47.6ml). To this, sodium azide (3.09 g, 47.6 mmol) was added and refluxed by heating for 11 hours. After the resultant was allowed to cool to room temperature, the reaction solution was concentrated, and the residue was dissolved in ethyl acetate . The organic layer was washed with 24 ml of a saturated aqueous solution of sodium chloride and dried over sodium sulfate. After the filtration, the filtrate was concentrated, and thereby a crude product was obtained. The crude product was purified by silica gel column chromatography (hexane/ethyl acetate=1/2 to 1/4). Thus, a colorless oily substance was obtained (compound 3, 2.80 g, 16.0 mmol, 67% yield) . The spectra were consistent with the values in the literature (Amaral, S. P. et al., Org. Lett. 2011, 13, 4522-4525).

### (ii) 2-(2-(2-Azidoethoxy)ethoxy)ethyl 4-methylbenzenesulfonate (Compound 4)

The compound 3 (2.80 g, 16.0 mmol) was dissolved in methylene chloride (32.0 ml), and p-toluenesulfonyl chloride (TsCl: 3.66 g, 19.2 mmol) and triethylamine (4.43 ml, 32.0 mmol) were added thereto and stirred at room temperature for 14 hours. Then, a 1 M aqueous solution of hydrochloric acid was added thereto, and the aqueous layer was extracted with methylene chloride. The organic layer thus recovered was washed with a saturated aqueous solution of sodium hydrogen carbonate and a saturated aqueous solution of sodium chloride, and dried over sodium sulfate. After the filtration, the filtrate was concentrated, and thereby a crude product was obtained. The crude product was purified by silica gel column chromatography (hexane/ethyl acetate=3/1 to 2/1). Thus, a colorless oily substance was obtained (compound 4, 4.92 g, 14.9 mmol, 93% yield) . The spectra were consistent with the values in the literature (Deng, L. et al., Org. Biomol. Chem. 2011, 9, 3188-3198).

### (iii) Methyl 3,4,5-tris(2-(2-(2-azidoethoxy)ethoxy)ethoxy)benzoate (Compound 5)

Methyl gallate (688 mg, 3.73 mmol) was dissolved in N,N-dimethylformamide (DMF: 94.0 ml), and potassium carbonate (5.16 g, 37.3 mmol) was added thereto. To this, the compound 4 (4.92 g, 14.9 mmol) dissolved in DMF (19.0 ml) was added, and then stirred at 80°C for 24 hours. After the resultant was allowed to cool to room temperature, the solid was removed by filtration, and the filtrate was concentrated. The residue was dissolved in t-butanol and then concentrated again. The residue was dissolved in methylene chloride. The organic layer was washed with water and a saturated aqueous solution of sodium chloride and dried over sodium sulfate. After the filtration, the filtrate was concentrated, and thereby a crude product was obtained (4.00g). The crude product was purified by silica gel column chromatography (hexane/ethyl acetate=1/1 to 1/4). Thus, a thin yellow oily substance was obtained (compound 5, 2.30 g, 3.51 mmol, 94% yield) . The spectra were consistent with the values in the literature (Amaral, S. P. et al., Org. Lett. 2011, 13, 4522-4525).

### (iv) 3,4,5-Tris(2-(2-(2-azidoethoxy)ethoxy)ethoxy)benzoic acid (Compound 6)

The compound 5 (1.82 g, 2.78 mmol) was dissolved in ethanol (77.3 ml), and a 1 M aqueous solution of potassium hydroxide (5.17 ml, 5.17 mmol) was added thereto and refluxed by heating for 2 hours. After the resultant was allowed to cool to room temperature, the reaction solution was distilled off. The residue was dissolved in methylene chloride, and a 1 M aqueous solution of hydrochloric acid was carefully added thereto until the aqueous layer showed an acidity (pH=1). The aqueous layer was extracted with methylene chloride, washed with a saturated aqueous solution of sodium chloride, and dried over sodium sulfate. After the filtration, the filtrate was concentrated, and thereby a crude product was obtained (compound 6, 1.67 g) . The obtained crude product was used in the subsequent reaction without purification.

### (v) Methyl(3,4,5-tris(2-(2-(2-azidoethoxy)ethoxy)ethoxy)be nzoyl)glycinate (Compound 7)

The crude product 6 (1.36g) was dissolved in methylene chloride (10.6ml), and glycine methyl ester hydrochloride (293 mg, 2.33 mmol), N-(3-dimethylaminopropyl)-N'-ethylcarbodiimide hydrochloride (WSC: 447 mg, 2.33 mmol), triethylamine (0.646 ml, 4.66 mmol), and 4-(dimethylamino)pyridine (DMAP: 25.9mg, 0.212 mmol) were added thereto, and stirred at room temperature for 22 hours. After a 1 M aqueous solution of hydrochloric acid was added, the aqueous layer was extracted with methylene chloride. The organic layer thus recovered was washed with a saturated aqueous solution of sodium hydrogen carbonate and a saturated aqueous solution of sodium chloride, and dried over sodium sulfate. After the filtration, the filtrate was concentrated, and thereby a crude product was obtained (1.72 g). The crude product was purified by silica gel column chromatography (methylene chloride/methanol=100/0to 13/1). Thus, a thin yellow oily substance was obtained (compound 7, 1.15 g, 1.61 mmol, 71% yield over two steps).
¹H NMR (CDCl₃, 500 MHz) δ 7.05 (s, 2H), 6.76 (t, J=5.2 Hz, 1H), 4.16-4.14 (m, 8H), 3.80 (t, J=5.2 Hz, 4H), 3.75 (t, J=5.2Hz, 2H), 3.73 (s, 3H), 3.68-3.66 (m, 6H), 3.62-3.59 (m, 12H), 3.32 (t, J=4.9 Hz, 6H); ¹³C NMR (CDCl₃, 125 MHz) δ 170.4, 166.9, 152.4, 141.6, 128.7, 107.0, 77.2, 72.3, 70.6, 70.6, 70.6, 70.5, 70.4, 69.9, 69.9, 69.7, 68.9, 52.3, 50.5, 41.6; ESI-MS m/z 735 [M+Na]⁺.

### (vi) (3,4,5-Tris(2-(2-(2-azidoethoxy)ethoxy)ethoxy)benzoyl) glycine (Compound 8)

The compound 7 (319 mg, 0.448 mmol) was dissolved in methanol (4.48 ml), and a 1 M aqueous solution of sodium hydroxide (0.895 ml, 0.895 mmol) was added thereto and stirred at room temperature for 90 minutes. Amberlyst 15-DRY was added thereto until the reaction solution became acidic (pH=4). After the filtration, the filtrate was concentrated, and thereby a crude product was obtained (compound 8, 297 mg). The obtained crude product was used in the subsequent reaction without purification.

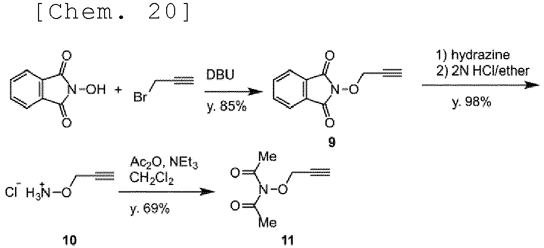

### (vii) 2-(Prop-2-yn-1-yloxy)isoindoline-1,3-dione (Compound 9)

Propargyl bromide (4.01 g, 33.7 mmol) was dissolved in DMF (76.6 ml), and N-hydroxyphthalimide (5.00 g, 30.7 mmol) and 1,8-diazabicyclo[5.4.0]undec-7-ene (DBU: 4.58 ml, 30.7 mmol) were added thereto and stirred at room temperature for 1 hour. The reaction solution was carefully poured into a 1 M aqueous solution of hydrochloric acid (768 ml) having been cooled on ice. The precipitate thus formed was collected by filtration, and washed with water, ethanol, and diethyl ether in this order. Thus, the target substance was obtained in the form of a white powder (compound 9, 5.27 g, 26.2 mmol, 85% yield). The spectra were consistent with the values in the literature (Kim, J. N. et al., Synth. Commun. 1992, 22, 1427-1432) .

### (viii) O-(Prop-2-yn-1-yl)hydroxylammonium chloride (Compound 10)

Hydrazine hydrate (1.44 g, 28.8 mmol) and the compound 9 (5.27 g, 26.2 mmol) were mixed and stirred at room temperature for 5 minutes. Then, diethyl ether (26.2 ml) was added and further stirred vigorously for 45 minutes. The precipitate thus formed was removed by filtration, and the resultant was washed with diethyl ether and then combined with the filtrate. 2 M hydrochloric acid (diethyl ether solution, 19.0 ml, 38.0 mmol) was added thereto while being stirred. The resultant was stirred at room temperature for 3 hours. The precipitate thus formed was collected by filtration, and washed with diethyl ether. Thus, the target substance was obtained in the form of a white solid (compound 10, 2.76 g, 25.6 mmol, 98% yield). The spectra were consistent with the values in the literature (Banerjee, D. et al., ChemBioChem 2010, 11, 1273-1279).

### (ix) N-Acetyl-N-(prop-2-yn-1-yloxy)acetamide (Compound 11)

The compound 10 (300 mg, 2.79 mmol) was dissolved in methylene chloride (2.79 ml) and cooled on ice. Then, triethylamine (1.55 ml, 11.2 mmol) and acetic anhydride (Ac₂O: 0.659 ml, 6.97 mmol) were slowly added thereto. Subsequently, the reaction solution was stirred at room temperature for 3 hours. Diethyl ether was added thereto, and the precipitate thus formed was removed by filtration. The filtrate was concentrated, and thereby a crude product was obtained (1.32 g). The crude product was purified by silica gel column chromatography (hexane/ethyl acetate=5/1 to 3/2). Thus, a colorless oily substance was obtained (compound 11, 298 mg, 1.92 mmol, 69% yield).
¹H NMR (CDCl₃, 500 MHz) δ 4.65 (d, J=2.4 Hz, 2H), 2.59 (t, J=2.4 Hz, 1H), 2.36 (s, 6H); ¹³C NMR (CDCl₃, 125 MHz) δ 170.1, 78.2, 76.4, 62.9, 24.9; ESI-MS m/z 178 [M+Na]⁺.

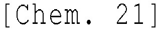

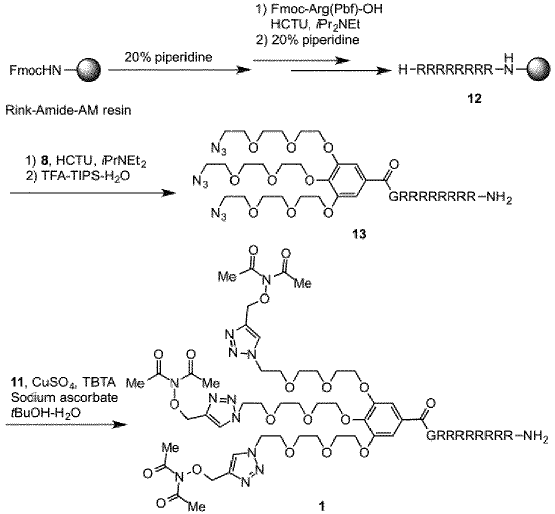

### (x) Azide Compound (Compound 13)

A Rink-Amide-AM resin (0.79 mmol/g, 100 mg, 0.079 mmol) was measured and put into a PD-10 column (GE Healthcare), and methylene chloride (3 ml) was added thereto and shaken for 10 hours, thereby allowing the resin to swell. The methylene chloride was removed by suction filtration, and the resultant was washed with DMF (3 ml) five times. Then, 20% piperidine (DMF solution, 2 ml) was added and shaken for 30 seconds. After the suction filtration, 20% piperidine (DMF solution, 2 ml) was further added and shaken for 10 minutes. After the suction filtration, the resultant was washed with DMF (3 ml) ten times. To this, Fmoc-Arg(Pbf)-OH (314 mg, 0.484 mmol, Fmoc: 9-fluorenylmethyloxycarbonyl, Pbf: 2,2,4,6,7-Pentamethyldihydrobenzofuran-5-sulfonyl chloride),
O-(6-Chlorobenzotriazol-1-yl)-N,N,N',N'-tetramethyluro nium hexafluorophosphate (HCTU: 189 mg, 0.457 mmol), DMF (2 ml), and iPr₂NEt (0.0790 ml, 0.453 mmol) were added and shaken for 1 hour. After the suction filtration, the resultant was washed with DMF (3 ml) five times. The removal of the Fmoc group with piperidine and the condensation of Fmoc-Arg (Pbf) -OH were repeated eight times. Finally, the removal of the Fmoc group with piperidine was performed one more time. Thus, a compound 12 was obtained. To this, HCTU (189 mg, 0.457 mmol), the compound 8 (331 mg, 0.474 mmol) dissolved in DMF (2 ml), and iPr₂NEt (0.0790 ml, 0.453 mmol) were added and shaken for 2 hours. After the suction filtration, the resultant was washed with DMF (3 ml) five times, and further washed with methanol (3 ml) five times. The resulting resin was placed into a vial, and water (0.125ml), triisopropylsilane (TIPS: 0.125ml), and trifluoroacetic acid (TFA: 4.75 ml) were added thereto and stirred at room temperature for 90 minutes. The resin was removed by filtration, and the filtrate was concentrated. Subsequently, diethyl ether (30 ml) was added to the residue, and the precipitate thus formed was collected by filtration. Thereby, a crude product was obtained. The crude product was purified by HPLC (ODS, linear gradient; 10-90% acetonitrile/0.1% TFA aqueous solution, 60 minutes, 254 nm, 10 ml/minute). Thus, the target substance was obtained in the form of a white solid (compound 13, 36.8mg). HPLC retention time : 27.6 minutes . MALDI-TOF MS m/z Calcd: 1947.13 [M+H]⁺, Found: 1947.47.

### (xi) Acetylating Agent (Compound 1)

The compound 13 (38.4mg) was dissolved in water (5.41 ml) and t-butanol (5.42 ml), and a t-butanol solution of the compound 11 (60mM, 1.97 ml, 0.118 mmol) was added thereto. To this, a separately-prepared 2 mM solution of a copper catalyst (4.92 ml, prepared by adding t-butanol (2.46 ml) and a 4 mM aqueous solution of CuSO₄ (2.46 ml) to tris[(1-benzyl-1H-1,2,3-triazol-4-yl)methyl]amine (TBTA) : 10.5 mg, 0.0198 mmol) and a 25 mM aqueous solution of sodium ascorbate (25 mM, 1.97 ml, 0.0493 mmol) were added and stirred at room temperature. Four hours later, the compound 11 (18.0 mg, 0.116 mmol), a 4 mM solution of a copper catalyst (2.46 ml, prepared by adding t-butanol (1.23 ml) and an 8 mM aqueous solution of CuSO₄ (1.23 ml) to TBTA: 10.5 mg, 0.0198 mmol), and sodium ascorbate (9.8 mg, 0.0495 mmol) were further added thereto and stirred at room temperature for 2 hours. The reaction solution was concentrated, and water (7 ml) was added thereto. After the insoluble matters were removed by filtration, the filtrate was purified by HPLC (ODS, linear gradient; 10-90% acetonitrile/0.1% TFA aqueous solution, 60 minutes, 254 nm, 10ml/minute). Thus, the target substance was obtained in the form of a white solid (compound 1, 36.7 mg). HPLC retention time: 26.3 minutes. MALDI-TOF MS (CHCA) m/z Calcd: 2412.31 [M+H]⁺, Found: 2412.01.

### (2) Synthesis of Chromosome-Localizable Malonylating Agent

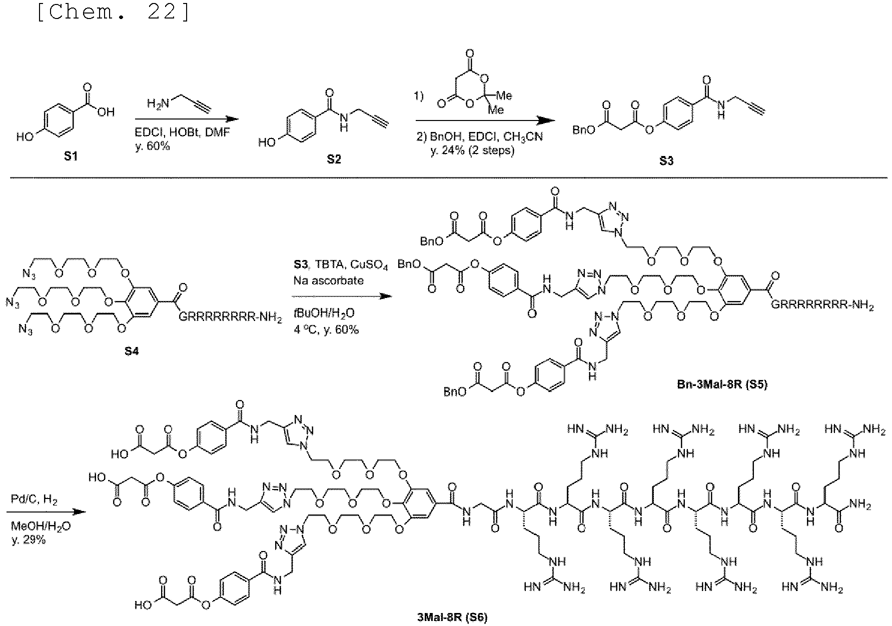

### (i) 4-Hydroxy-N-(prop-2-yn-1-yl)benzamide (Compound S2)

4-Hydroxybenzoic acid (S1, 1.0 g, 7.2 mmol) was dissolved in DMF (5 ml), and N-(3-dimethylaminopropyl)-N'-ethylcarbodiimide hydrochloride (EDCI, 1.7 g, 8.9 mmol), HOBT (0.5 g, 4.7 mmol), and propargylamine (0.5 ml, 7.2 mmol) were added thereto and stirred overnight. The solvent was distilled off, and thereby a crude product was obtained. Then, the crude product was purified by silica gel column chromatography (chloroform/acetone=8/1 to 4/1). Thus, the target substance was obtained in the form of a pale yellow solid (S2, 753.6 mg, 60% yield).
¹H NMR (CD₃OD, 400 MHz) δ 7.69 (d, J=9.1 Hz, 2H), 6.81 (d, J=9.1 Hz, 2H), 4.11 (d, J=2.5 Hz, 2H), 2.55 (t, J=2.5 Hz, 1H).

### (ii) Benzyl(4-(prop-2-yn-1-ylcarbamoyl)phenyl)malonate (Compound S3)

The compound S2 (175.2 mg, 1.0 mmol) was mixed with 2,2-dimethyl-1,3-dioxane-4,6-dione (144.1 mg, 1.0mmol), and stirred at 100°C for 2 hours. After the resultant was allowed to cool to room temperature, acetonitrile (5 ml), benzyl alcohol (360 µl, 3.0 mmol), and EDCI (372.0 mg, 2.0 mmol) were added and stirred at room temperature for 8 hours . After the solvent was distilled off, the residue was dissolved in ethyl acetate, and the organic layer was washed with a saturated aqueous solution of sodium chloride, and dried over sodium sulfate. After the filtration, the filtrate was concentrated, and thereby a crude product was obtained. The crude product was purified by HPLC (YMC-Pack ODS-AM (20 mm I.D.x250) column, linear gradient; 1-90% acetonitrile/0.1% TFA aqueous solution, 60 minutes, 230 nm, 10mL/minute) . Thus, the target substance was obtained in the form of a yellow solid (S3, 82.0 mg, 0.23 mmol, 23% yield). HPLC retention time: 30.0 minutes.
¹H NMR (CDCl₃, 400 MHz) δ 7.77 (d, J=8.7 Hz, 2H), 7.36 (s, 5H), 7.12 (d, J=8.7 Hz, 2H), 6.16 (s, 1H), 5.23 (s, 2H), 4.22-4.25 (dd, J=2.8, 5.5 Hz, 2H), 3.65 (s, 2H), 2.27 (t, J=2.8 Hz, 1H).

### (iii) Bn-3Mal-8R (Compound S5)

A compound S4 (16.0 mg, 0.0056 mmol) was dissolved in water-tBuOH (1:1, 5.6ml), and an acetonitrile solution of the alkyne S3 (160 mM, 0.29 ml, 0.0448 mmol), a separately-prepared water-tBuOH solution of Cu-TBTA (CuSO₄: TBTA=1:1, 1 mM, 0.626 ml, 0.0056 mmol), and an aqueous solution of ascorbic acid (50 mM, 0.56 ml, 0.0280 mmol) were added thereto and stirred at 4°C for 1 hour. Then, the resultant was purified by HPLC (YMC-Pack ODS-AM (20 mm I.D.x250) column, linear gradient; 30-50% acetonitrile/0.1% TFA aqueous solution, 60 minutes, 230 nm, 10 mL/minute). After the freeze-drying, the target substance was obtained in the form of a white solid (S5, 13.13 mg, 60% yield) . HPLC retention time: 28.0 minutes.

### (iv) 3Mal-8R (Compound S6)

Bn-3Mal-8R (S5, 1.9 mg, 0.00049 mmol) was dissolved in water-methanol (3:1, 0.8 ml), and 10% Pd/C (0.6 mg) was added thereto and stirred in a hydrogen atmosphere at room temperature for 1 hour. Then, the reaction solution was filtered through Celite, and the filtrate was concentrated. Thereby, a crude product was obtained. The crude product was purified by HPLC (YMC-Pack ODS-AM (10 mm I.D.x250) column, linear gradient; 10-30% acetonitrile/0.1% TFA aqueous solution, 60 minutes, 230 nm, 3 mL/minute) . After the freeze-drying, the target substance was obtained in the form of a white solid (S6, 0.51 mg, 29% yield).
MALDI-TOFMS (CHCA) m/z Calcd: 2731.33 [M+H]⁺, Found: 2731.56, HPLC retention time: 38.0 minutes.

### (Synthesis Example 2) Synthesis of Chromosome-Localizable Nucleophilic Catalyst

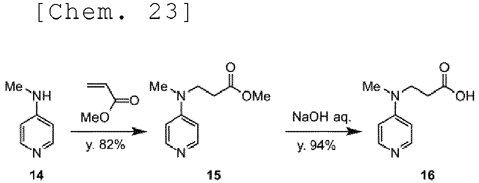

### (i) Methyl 3-(methyl-4-pyridylamino)propionate (Compound 15)

Into a flask, methyl acrylate (9.00 ml, 99.9 mmol) and 4-(methylamino)pyridine (compound 14, 541 mg, 5.00 mmol) were added and refluxed at 85°C for 19 hours. The solvent was distilled off under reduced pressure, and the residue was purified by silica gel column chromatography (methylene chloride /methanol=15/1 to 10/1). Thus, the target substance was obtained in the form of a pale yellow oily compound (compound 15, 800mg, 4.12 mmol, 82% yield) . The spectra were consistent with the values in the literature (Bhattacharya, S. & Snehalatha, K. J. Chem. Soc. , Perkin Trans. 2 1996, 2021-2025).

### (ii) 3-(Methyl-4-pyridylamino)propionic acid (Compound 16)

Into a flask, methyl 3-(methyl-4-pyridylamino) propionate (compound 15, 777 mg, 4.00 mmol), methanol (5.00 ml), and a 2 M aqueous solution of sodium hydroxide (5.00 ml, 10.0 mmol) were added and stirred at room temperature for 9 hours. 1 N hydrochloric acid (7 ml) was added thereto to adjust the pH to 7. Then, the solvent was distilled off under reduced pressure, and the residue was purified by silica gel column chromatography (methylene chloride/methanol = 1/1 to 2/3) . Thus, the target substance was obtained in the form of a white solid (compound 16, 680 mg, 3.78 mmol, 94% yield). The spectra were consistent with the values in the literature (Bhattacharya, S. & Snehalatha, K. J. Chem. Soc., Perkin Trans. 2 1996, 2021-2025).

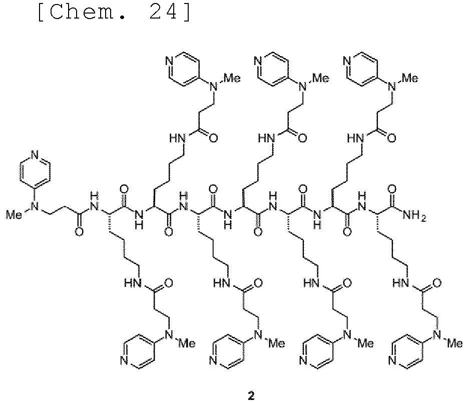

### (iii) L-8DMAP (Compound 2)

A Rink Amide AM resin (0.79 mmol/g, 100 mg, 0.079 mmol) was measured and put into a PD-10 column (GE Healthcare), and allowed to swell with DMF. A DMF solution of 20% piperidine was added thereto and stirred for 10 minutes to remove the Fmoc group. The resultant was washed with DMF eight times. Then, 5 equivalents of each of Fmoc-L-Lys(Mtt)-OH (247 mg, 0.395 mmol, Mtt: 4-methyltrityl), 1-hydroxybenzotriazole (HOBt: 60.5 mg, 0.395mmol), N,N'-diisopropylcarbodiimide (DIC: 0.0610ml, 0.395 mmol), and DMF (3 ml) were added thereto, stirred at room temperature for 1 hour, and subjected to an amino-acid coupling reaction. Similarly, the operations of the Fmoc group removal and the coupling reaction were performed seven times in total. Thereby, hepta-L-Lys (Mtt) was prepared on the resin. After washing with DMF, the resultant was further washed with dichloromethane five times, and the solvent was replaced. A dichloromethane solution of 3% TFA and 5% TIPS was added, and 30-second stirring was repeated 12 times. Thereby, the Mtt group was removed. After the resultant was washed with dichloromethane, the solvent was replaced with DMF. The compound 16 (284 mg, 1.58 mmol), HOBt (242 mg, 1.58 mmol), DIC (0.247 ml, 1.58 mmol), and DMF (3 ml) were each added in an amount of 2.5 equivalents relative to an amino acid of the lysine residue, and stirred at room temperature for 15 hours . After washing with DMF, the solvent was replaced with methanol, and the resin was vacuum-dried. The resulting resin was placed in a vial, and a TFA solution of 2.5% TIPS and 2.5% water was added thereto and stirred for 1 hour. Thereby, the peptide was cleaved from the resin. The solvent was distilled off under reduced pressure, and diethyl ether was added to the resultant in an ice bath. The precipitate thus obtained was collected by filtration. Thereby, a crude product was obtained. The crude product was purified by HPLC (ODS, linear gradient; 10-100% acetonitrile/0.1% TFA aqueous solution, 40 minutes, 230 nm, 10 ml/minute) and freeze-dried. Thus, the target substance was obtained in the form of a white solid (2, 42.0 mg). HPLC retention time: 15.3 minutes. MALDI-TOF MS (CHCA): m/z Calcd: 2211.33 [M+H]⁺, Found: 2211.59.

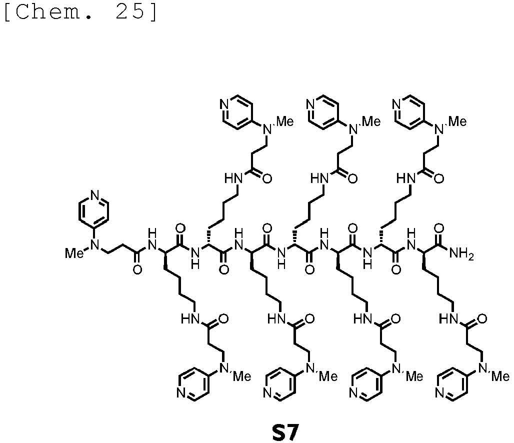

### (iv) D-8DMAP (Compound S7) :

A NovaPEG Rink Amide resin (0.45 mmol/g, 222 mg, 0.10 mmol) was measured and put into a PD-10 column (GE Healthcare), and allowed to swell with DMF. A DMF solution of 20% piperidine was added thereto and stirred for 10 minutes to remove the Fmoc group. The resultant was washed with DMF ten times. Then, 4 equivalents of each of Fmoc-D-Lys (Mtt) -OH (250 mg, 0.40 mmol), HOBt (61.3 mg, 0.40 mmol), DIC (61.9 µL, 0.40 mmol), and DMF (3 mL) were added thereto, stirred at room temperature for 1 hour, and subjected to an amino-acid coupling reaction. Similarly, the operations of the Fmoc group removal and the coupling reaction were performed seven times in total. Thereby, hepta-D-Lys (Mtt) was prepared on the resin. After washing with DMF, the resultant was further washed with dichloromethane five times, and the solvent was replaced. A dichloromethane solution of 3% TFA and 5% TIPS was added, and 30-second stirring was repeated 12 times. Thereby, the Mtt group was removed. After the resultant was washed with dichloromethane, the solvent was replaced with DMF. The compound 16 (360 mg, 2.0 mmol), HOBt (306 mg, 2.0 mmol), DIC (310 µL, 2.0 mmol), and DMF (3 mL) were each added in an amount of 2.5 equivalents relative to an amino acid of the lysine residue, and stirred at room temperature for 15 hours . After washing with DMF, the solvent was replaced with methanol, and the resin was vacuum-dried. The resulting resin was placed in a vial, and a TFA solution of 2.5% TIPS and 2.5% water was added thereto and stirred for 1 hour. Thereby, the peptide was cleaved from the resin. The solvent was distilled off under reduced pressure, and diethyl ether was added to the resultant. The precipitate thus obtained was collected by filtration. Thereby, a crude product was obtained. The crude product was purified by HPLC (Triart column, linear gradient; 0-100% acetonitrile/0.1% TFA aqueous solution, 100 minutes, 230 nm, 3 mL/minute) and freeze-dried. Thus, the target substance was obtained in the form of a white solid (S7, 45.4mg). Reversed-phase HPLC retention time=15.1 minutes (ODS column, 4.6x150 mm, linear gradient; 0-100% acetonitrile/0.1% TFA aqueous solution, 40 minutes, 230 nm, 0.9 ml/minute). MS (ESI) : m/z calcd: 553.59 [M+4H]⁴⁺, found: 553.59.

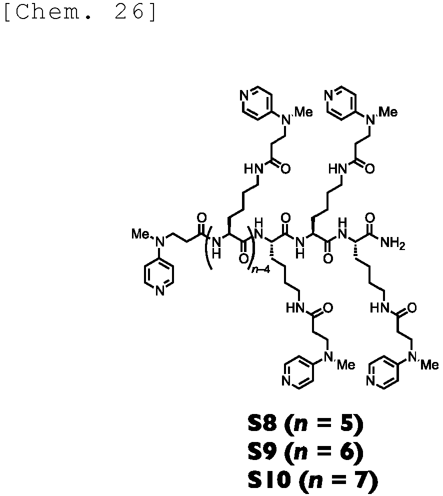

### (v) L-5DMAP (Compound S8) :

A NovaPEG Rink Amide resin (0.45 mmol/g, 222 mg, 0.10 mmol) was measured and put into a PD-10 column (GE Healthcare), and allowed to swell with DMF. A DMF solution of 20% piperidine was added thereto and stirred for 10 minutes to remove the Fmoc group. The resultant was washed with DMF ten times. Then, 4 equivalents of each of Fmoc-L-Lys (Mtt) -OH (250 mg, 0.40 mmol), HOBt (61.3mg, 0.40 mmol), DIC (61.9 µL, 0.40 mmol), and DMF (3 mL) were added thereto, stirred at room temperature for 1 hour, and subjected to an amino-acid coupling reaction. Similarly, the operations of the Fmoc group removal and the coupling reaction were performed four times in total. Thereby, tetra-L-Lys (Mtt) was prepared on the resin. After washing with DMF, the resultant was further washed with dichloromethane five times, and the solvent was replaced. A dichloromethane solution of 3% TFA and 5% TIPS was added, and 30-second stirring was repeated 12 times. Thereby, the Mtt group was removed. After the resultant was washed with dichloromethane, the solvent was replaced with DMF. The compound 16 (225 mg, 1.25 mmol), HOBt (191 mg, 1.25 mmol), DIC (193 µL, 1.25 mmol), and DMF (3 mL) were each added in an amount of 2.5 equivalents relative to an amino acid of the lysine residue, and stirred at room temperature for 15 hours. After washing with DMF, the solvent was replaced with methanol, and the resin was vacuum-dried. The resulting resin was placed in a vial, and a TFA solution of 2.5% TIPS and 2.5% water was added thereto and stirred for 1 hour. Thereby, the peptide was cleaved from the resin. The solvent was distilled off under reduced pressure, and diethyl ether was added to the resultant. The precipitate thus obtained was collected by filtration. Thereby, a crude product was obtained. The crude product was purified by HPLC (Triart column, linear gradient; 0-100% acetonitrile/0.1% TFA aqueous solution, 100 minutes, 230 nm, 3 mL/minute) and freeze-dried. Thus, the target substance was obtained in the form of a white solid (S8, 35.2mg). Reversed-phase HPLC retention time=14.3 minutes (ODS column, 4.6x150 mm, linear gradient; 0-100% acetonitrile/0.1% TFA aqueous solution, 40 minutes, 230 nm, 0.9 ml/minute) . MS (ESI) : m/z calcd: 447.61 [M+3H]³⁺, found: 447.53.

### (vi) L-6DMAP (Compound S9) :

A NovaPEG Rink Amide resin (0.45 mmol/g, 222 mg, 0.10 mmol) was measured and put into a PD-10 column (GE Healthcare), and allowed to swell with DMF. A DMF solution of 20% piperidine was added thereto and stirred for 10 minutes to remove the Fmoc group. The resultant was washed with DMF ten times. Then, 4 equivalents of each of Fmoc-L-Lys(Mtt)-OH (250 mg, 0.40 mmol), HOBt (61.3 mg, 0.40 mmol), DIC (61.9 µL, 0.40 mmol), and DMF (3 mL) were added thereto, stirred at room temperature for 1 hour, and subjected to an amino-acid coupling reaction. Similarly, the operations of the Fmoc group removal and the coupling reaction were performed five times in total. Thereby, penta-L-Lys (Mtt) was prepared on the resin. After washing with DMF, the resultant was further washed with dichloromethane five times, and the solvent was replaced. A dichloromethane solution of 3% TFA and 5% TIPS was added, and 30-second stirring was repeated 12 times. Thereby, the Mtt group was removed. After the resultant was washed with dichloromethane, the solvent was replaced with DMF. The compound 16 (270 mg, 1.50 mmol), HOBt (229 mg, 1.50 mmol), DIC (232 µL, 1.50 mmol), and DMF (3 mL) were each added in an amount of 2.5 equivalents relative to an amino acid of the lysine residue, and stirred at room temperature for 15 hours. After washing with DMF, the solvent was replaced with methanol, and the resin was vacuum-dried. The resulting resin was placed in a vial, and a TFA solution of 2.5% TIPS and 2.5% water was added thereto and stirred for 1 hour. Thereby, the peptide was cleaved from the resin. The solvent was distilled off under reduced pressure, and diethyl ether was added to the resultant. The precipitate thus obtained was collected by filtration. Thereby, a crude product was obtained. The crude product was purified by HPLC (Triart column, linear gradient; 0-100% acetonitrile/0.1% TFA aqueous solution, 100 minutes, 230 nm, 3 mL/minute) and freeze-dried. Thus, the target substance was obtained in the form of a white solid (S9, 57.8mg). Reversed-phase HPLC retention time=14.5 minutes (ODS column, 4.6x150 mm, linear gradient; 0-100% acetonitrile/0.1% TFA aqueous solution, 40 minutes, 230 nm, 0.9 ml/minute) . MS (ESI) : m/z calcd: 408.50 [M+4H]⁴⁺, found: 408.43.

### (vii) L-7DMAP (Compound S10) :

A NovaPEG Rink Amide resin (0.45 mmol/g, 222 mg, 0.10 mmol) was measured and put into a PD-10 column (GE Healthcare), and allowed to swell with DMF. A DMF solution of 20% piperidine was added thereto and stirred for 10 minutes to remove the Fmoc group. The resultant was washed with DMF ten times. Then, 4 equivalents of each of Fmoc-L-Lys (Mtt) -OH (250 mg, 0.40 mmol), HOBt (61.3 mg, 0.40 mmol), DIC (61.9 µL, 0.40 mmol), and DMF (3 mL) were added thereto, stirred at room temperature for 1 hour, and subjected to an amino-acid coupling reaction. Similarly, the operations of the Fmoc group removal and the coupling reaction were performed six times in total. Thereby, hexa-L-Lys (Mtt) was prepared on the resin. After washing with DMF, the resultant was further washed with dichloromethane five times, and the solvent was replaced. A dichloromethane solution of 3% TFA and 5% TIPS was added, and 30-second stirring was repeated 12 times. Thereby, the Mtt group was removed. After the resultant was washed with dichloromethane, the solvent was replaced with DMF. The compound 16 (315 mg, 1.75 mmol), HOBt (256 mg, 1.75 mmol), DIC (271 µL, 1.75 mmol), and DMF (3 mL) were each added in an amount of 2.5 equivalents relative to an amino acid of the lysine residue, and stirred at room temperature for 15 hours. After washing with DMF, the solvent was replaced with methanol, and the resin was vacuum-dried. The resulting resin was placed in a vial, and a TFA solution of 2.5% TIPS and 2.5% water was added thereto and stirred for 1 hour. Thereby, the peptide was cleaved from the resin. The solvent was distilled off under reduced pressure, and diethyl ether was added to the resultant. The precipitate thus obtained was collected by filtration. Thereby, a crude product was obtained. The crude product was purified by HPLC (Triart column, linear gradient; 0-100% acetonitrile/0.1% TFA aqueous solution, 100 minutes, 230 nm, 3 mL/minute) and freeze-dried. Thus, the target substance was obtained in the form of a white solid (S10, 57.6mg). Reversed-phase HPLC retention time=14.6 minutes (ODS column, 4.6x150 mm, linear gradient; 0-100% acetonitrile/0.1% TFA aqueous solution, 40 minutes, 230 nm, 0.9 ml/minute). MS (ESI) : m/z calcd: 481.05 [M+4H]⁴⁺, found: 480.96.

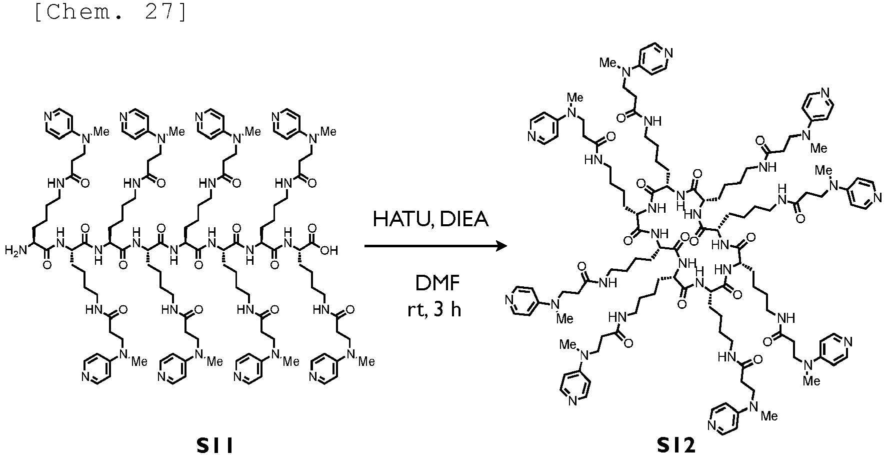

### (viii) H-8DMAP-OH (Compound S11)

A Fmoc-L-Lys(Mtt)-Alko-PEG resin (0.22 mmol/g, 455 mg, 0.10 mmol) was measured and put into a PD-10 column (GE Healthcare), and allowed to swell with DMF. A DMF solution of 20% piperidine was added thereto and stirred for 10 minutes to remove the Fmoc group. The resultant was washed with DMF ten times . Then, 4 equivalents of each of Fmoc-L-Lys (Mtt)-OH (250 mg, 0.40 mmol), HOBt (61.3 mg, 0.40 mmol), DIC (61.9 µL, 0.40 mmol), and DMF (3 mL) were added thereto, stirred at room temperature for 1 hour, and subjected to an amino-acid coupling reaction. Similarly, the operations of the Fmoc group removal and the coupling reaction were performed six times in total. Thereby, H-octa-L-Lys(Mtt) was prepared on the resin. After washing with DMF, the resultant was further washed with dichloromethane five times, and the solvent was replaced. A dichloromethane solution of 3% TFA and 5% TIPS was added, and 30-second stirring was repeated 12 times. Thereby, the Mtt group was removed. After the resultant was washed with dichloromethane, the solvent was replaced with DMF. The compound 16 (360 mg, 2.0mmol), HOBt (306 mg, 2.0 mmol), DIC (310 µL, 2.0 mmol), and DMF (3 mL) were each added in an amount of 2.5 equivalents relative to an amino acid of the lysine residue, and stirred at room temperature for 15 hours . After washing with DMF, the solvent was replaced with methanol, and the resin was vacuum-dried. The resulting resin was placed in a vial, and a TFA solution of 2.5% TIPS and 2.5% water was added thereto and stirred for 1 hour. Thereby, the peptide was cleaved from the resin. The solvent was distilled off under reduced pressure, and diethyl ether was added to the resultant. The precipitate thus obtained was collected by filtration. Thereby, a crude product was obtained. The crude product was purified by HPLC (Triart column, linear gradient; 0-100% acetonitrile/0.1% TFA aqueous solution, 100 minutes, 230 nm, 3 mL/minute) and freeze-dried. Thus, the target substance was obtained in the form of a white solid (S11, 192 mg) . Reversed-phase HPLC retention time=14.6 minutes (ODS column, 4.6x150 mm, linear gradient; 0-100% acetonitrile/0.1% TFA aqueous solution, 40 minutes, 230 nm, 0.9 ml/minute). MS (ESI) : m/z calcd: 585.86 [M+4H]⁴⁺, found: 585.64.

### (ix) cyclo-L-8DMAP (Compound S12)

The compound S11 (163 mg, 50 µmol) and HATU (38 mg, 100 µmol) were dissolved in DMF (100 mL). Then, DIEA (35 µL, 200 µmol) was added thereto, and the reaction solution was stirred at room temperature for 3 hours. After a 0.1% aqueous solution of TFA (2 mL) was added thereto, the solvent was distilled off under reduced pressure. The resultant was dissolved again in a 0.1% aqueous solution of TFA (5 mL), purified by HPLC (Triart column, linear gradient; 0-100% acetonitrile/0.1% TFA aqueous solution, 100 minutes, 230 nm, 3 mL/minute), and freeze-dried. Thus, the target substance was obtained in the form of a white solid (S12, 100 mg). Yield: 62%. Reversed-phase HPLC retention time=15.3 minutes (ODS column, 4.6x150 mm, linear gradient; 0-100% acetonitrile/0.1% TFA aqueous solution, 40 minutes, 230 nm, 0.9 ml/minute). MS (ESI) : m/z calcd: 581.36 [M+4H]⁴⁺, found: 581.12.

### (Example 1) Chromosome Protein Acetylation with Chromosome-Localizable Acetylating Agent and Chromosome-Localizable Nucleophilic Catalyst, as well as Cancer-Cell Specific Cell Cycle Arrest and Analysis on Action Mechanism Thereof

### (1) Materials and Methods

### (i) Cell Culturing and Cell Fractionation

Hela cells and MCF7 cells were cultured using Dulbecco's modified Eagle's Medium (DMEM) media supplemented with 10% fetal bovine serum, 100 U/ml of penicillin, and 100 U/ml of streptomycin. The cells were cultured at 37°C in the presence of 5% CO₂.

The cell fractionation was carried out as follows. Approximately 10⁶ cells were detached from a culture dish by a trypsin treatment. After washed with PBS, the cell pellets were suspended in a cooled, cell lysis buffer [50 mM Tris (pH=7.5), 300 mM NaCl, 0.3% Triton X-100, protease inhibitor cocktail, and 1 mM PMSF], and placed on ice for 30 minutes. After the centrifugation (4°C, 1500 rpm, 2 minutes), the supernatant was collected as a cytoplasm fraction. Meanwhile, the pellets were suspended in a cell lysis buffer containing 2 mM MgCl₂ and Benzonase (Novagen, 71206), and placed on ice for 30 minutes. Then, 4 mM EDTA was added thereto to stop the reaction. After the centrifugation (4°C, 15000 rpm, 15 minutes), the supernatant was collected as a chromosome fraction.

### (ii) Western Blotting

Proteins were separated on a 4-20% SDS-PAGE gel, and transferred to a PVDF membrane, followed by blocking with 5% skim milk suspended in TBST for the reaction between the PVDF membrane and primary antibodies. The primary antibodies used were as follows. Anti-Histone H3 (acetyl K122) antibody (ab33309, Abcam), Acetylated-Lysine antibody (#9441, Cell Signaling), Anti-Histone H3 (acetyl K56) antibody (ab76307, Abcam), Anti-acetyl Histone H3 (Lys115) (#07-934, Millipore), Anti-P53 (DO-1) (sc-126, Santa cruz), Anti-Histone H3 (acetyl K9) antibody (ab4441, Abcam), Anti-Histone H3 (acetyl K14) antibody (ab52946, Abcam), Anti-Acetyl-Histone H4 (Ac-Lys16) (H9164, Sigma), Anti-Acetyl-Histone H4 (Ac-Lys5) (SAB4500307, Sigma), anti-acetyl tubulin (T6793, Sigma), Anti-p21/WAF1/Cip1 Antibody (05-655, Millipore), Anti-p27 (Kip1) (04-240, Millipore). After washing with TBST, the PVDF membrane was treated with a chemilluminescent detection reagent Luminata Forte Western HRP Substrate (Millipore), and the detection was performed using ImageQuant LAS 4000 (GE healthcare life sciences).

### (iii) Fluorescence Microscope

After compounds having FITC were added to the cells adhering on a glass coverslip, the cells were cultured, washed with PBS, fixed with 3.7 % formaldehyde for 10 minutes, and washed with PBS twice. Then, the cells were observed with a fluorescence microscope (Axioplan2; Carl Zeiss), and the photographic data were obtained.

### (iv) RNAi Transfection

Using Lipofectamine^{(R)} RNAiMAX Transfection Reagent (Invitrogen), siRNAs of CBP, p300, p53, and GL2 as a control were added each at a final concentration of 5 nM to the MCF-7 or Hela cells in a 25% confluent state. The cells were cultured at 37°C for 72 hours. The sequences of the siRNAs are shown below.
CBP-f: 5'-CGGCACAGCCUCUCAGUCATT-3' (SEQ ID NO: 1)
CBP-r: 5'-UGACUGAGAGGCUGUGCCGTT-3' (SEQ ID NO: 2)
p300-f: 5'-UGACACAGGCAGGCUUGACUUTT-3' (SEQ ID NO: 3)
p300-r: 5'-AAGUCAAGCCUGCCUGUGUCATT-3' (SEQ ID NO: 4)
hp53#1-f: 5'-GACUCCAGUGGUAAUCUACTT-3' (SEQ ID NO: 5)
hp53#1-r: 5'-GUAGAUUACCACUGGAGUCTT-3' (SEQ ID NO: 6)
hp53#2-f: 5'-CUACUUCCUGAAAACAACGTT-3' (SEQ ID NO: 7)
hp53#2-r: 5'-CGUUGUUUUCAGGAAGUAGTT-3' (SEQ ID NO: 8)
GL2-f: 5'-CGUACGCGGAAUACUUCGATT-3' (SEQ ID NO: 9)
GL2-r: 5'-UCGAAGUAUUCCGCGUACGTT-3' (SEQ ID NO: 10)

### (v) RNA Extraction and RNA Quantification by Real-Time PCR Method

Using RNeasy MinElute Cleanup Kit (Qiagen), RNA was extracted from each cell type. For the cDNA synthesis, 2 µg of the RNA and (Oligo (dT)₂₀) were used. The real-time PCR reaction was carried out using LightCycler 480 SYBR Green I Master System (Roche) and LightCycler480 System II (Roche). A housekeeping gene Actin was used as an internal control. The expression data were normalized with respect to Actin, and analyzed by using the 2^{-ΔΔCT} method (Livak KJ & Schmittgen TD (2001) Methods 25: 402-408). Three independent experiments were performed. In each experiment, mean values of three wells were calculated. The sequences of the primers used are shown below.
p53-f: 5'-CCCAAGCAATGGATGATTTGA-3' (SEQ ID NO: 11)
p53-r: 5'-GGCATTCTGGGAGCTTCATCT-3' (SEQ ID NO: 12)
p21-f: 5'-GCGATGGAACTTCGACTTTGT-3' (SEQ ID NO: 13)
p21-r: 5'-GGGCTTCCTCTTGGAGAAGAT-3' (SEQ ID NO: 14)
p27-f: 5'-AGACGGGGTTAGCGGAGCAA-3' (SEQ ID NO: 15)
p27-r: 5'-TCTTGGGCGTCTGCTCCACA-3' (SEQ ID NO: 16)
GAPDH-f: 5'-GGTGAAGGTCGGAGTCAACG-3' (SEQ ID NO: 17)
GAPDH-r: 5'-TGGGTGGAATCATATTGGAACA-3' (SEQ ID NO: 18)
Actin-f: 5'-TTGCCGACAGGATGCAGAA-3' (SEQ ID NO: 19)
Actin-r: 5'-GCCGATCCACACGGAGTACT-3' (SEQ ID NO: 20)

### (vi) Cell Cycle Analysis

The compounds were added to the cells having been cultured in the DMEM medium to be in 60-70% confluent states, and the cells were cultured for 15 to 22 hours. The samples were adjusted using BD cycle test plus DNA Reagent kit (BD Biosciences), and analyzed with a flow cytometer.

### (2) Result

First of all, the examination was conducted using: 4-dimethylaminopyridine (DMAP), widely used as a nucleophilic catalyst, as a catalyst; and N-methoxydiacetamide (NMD), known to acetylate a primary amine in water under mild conditions, as an acetylating agent (Yasuo Kikugawa, et al., Tetrahedron Letters 1990, 31, 243-246). A cell extract (nucleus fraction) of the human breast cancer MCF7 cells was mixed with these compounds, and allowed to react at room temperature for 12 hours. As a result, the acetylation of lysine residues of histones H3 and H4 was promoted (Fig. 1A, B). Next, after the MCF7 cells were treated with L-DMAP and NMD for 3 hours, the cell extract was collected and fractionated into a chromosome fraction (Chromatin) and a cytoplasm fraction (Cytoplasm) to then evaluate the protein acetylation levels by western blotting. As a result, a protein group contained in the cytoplasm fraction was highly acetylated, whereas there was no change in the protein acetylation in the chromosome fraction containing histones (Fig. 1C). This result revealed that L-DMAP and NMD were capable of enhancing the acetylation of lysine residues of proteins in cultured human cells . On the other hand, since the acetylation of the chromosome proteins including histones was not sufficiently enhanced, this suggested a possibility that L-DMAP or NMD was hardly accessible to the vicinity of chromosomes in a cell.

To examine the intracellular localization of DMAP, a compound was synthesized by fusing fluorescein isothiocyanate (FITC) to L-DMAP (Fig. 2A). As a result, the L-DMAP-FITC signal was mainly accumulated in the cytoplasm, and the accumulation was not observed in the nucleus and the chromosomes (Fig. 2B, C). A cluster of lysine and arginine, which are basic amino acids, has been used as a cell membrane-permeable peptide (Mitchell, D. J. et al., The journal of peptide research: official journal of the American Peptide Society 2000, 56, 318-325), but the localization to chromosomes has not been known. The examination was conducted to see whether or not a cluster of L-DMAP, which is a catalyst having a basic nature, as well, exhibited a cell membrane permeability, and also whether or not the cluster exhibited a chromosome-localizable activity. To be more specific, compounds were synthesized by fusing FITC to 3, 5, 6, 7, or 8 molecules of L-DMAP to examine the intracellular localization in the human cervical cancer HeLa cells or theMCF7 cells (Fig. 2A). As a result, the larger the number of L-DMAP, the more significantly the chromosome localization was observed. L-3DMAP-FITC was partially localized to the chromosomes, while L-5DMAP-FITC and the compounds having a larger number of L-DMAP were almost completely localized (Fig. 2B, C).

Next, the Hela cells were treated with L-8DMAP and NMD for 3 hours, and then the protein acetylation levels in the cells were evaluated as described above. Nevertheless, there was no change in the protein acetylation in the chromosome fraction containing histones (Fig. 2D).

Next, to examine the intracellular localization of NMD, a compound was synthesized by fusing FITC to 3NMD (Fig. 3A). As a result, the 3NMD-FITC signal was uniformly present in the entire cell, and the accumulation was not observed in the nucleus and the chromosomes (Fig. 3C). It has been known that 8-arginine (8R) is a cell membrane-permeable peptide (Mitchell, D. J. et al., The journal of peptide research: official journal of the American Peptide Society 2000, 56, 318-325) . Since 8R has a polycationic structure in water like 8DMAP, the accumulation to chromosomes was predicted. 8R-FITC was synthesized by fusing FITC to 8R to examine the intracellular localization in the HeLa cells . As a result, 8R-FITC was localized in the nucleus and the chromosomes (Fig. 3B). In order to localize 3NMD to the chromosomes, a compound was synthesized by fusing 3NMD to 8R to examine the intracellular localization in the MCF7 cells. As a result, the compound was successfully localized in the nucleus and the chromosomes as predicted (Fig. 3C). From these results, 3NMD-8R was used as a chromosome-localizable acetylating agent hereinafter.

The chromosome-localizable catalyst 8DMAP and the chromosome-localizable acetylating agent 3NMD-8R (Fig. 4A) developed above were administered to the MCF7 cells, followed by culturing for 3 hours. Then, protein acetylation levels in the cells were evaluated as described above. As a result, a protein group contained in the cytoplasm fraction was hardly acetylated, whereas proteins in the chromosome fraction containing histones were highly acetylated (Fig. 4B) . Using an acetylation-specific antibody against lysines present in an N-terminal tail region and a globular region (globular domain) of histone H3, each acetylation level was examined. As a result, there was no change in the acetylation of K9 and K14 present in the N-terminal tail region, whereas the acetylations of K56, K115 and K122 present in the H3 globular region were significantly enhanced (Fig. 4B). In addition, there was no change in the acetylation of K5 and K16 present in an N-terminal tail region of histone H4, either (Fig. 4B).

Note that the acetylation of K122 was examined using D-8DMAP, L-nDMAP (n=5 to 7), or cyclo-L-8DMAP as a chromosome-localizable catalyst in place of L-8DMAP. As a result, the acetylation levels were similar to that by L-8DMAP.

According to the above results, the combination of the chromosome-localizable catalyst and the chromosome-localizable acetylating agent successfully enhanced selective acetylation of chromosome proteins including histones in the cells.

It was predicted that the acetylation enhanced in the cells was due to a chemical reaction between the catalyst and the acetylating agent thus administered, and not dependent on acetyltransferases in the cells. To examine this possibility, 8DMAP and 3NMD-8R were administered to MCF7 cells and HeLa cells whose CBP and p300, which are main histone acetyltransferases (HAT) in cells (Ogryzko, V. V. et al., Cell 1996, 87, 953-959, Bannister, A. J. & Kouzarides, T. Nature 1996, 384, 641-643), had been knocked down by the RNAi method. After the cells were cultured for 3 hours, the protein acetylation levels in the cells were evaluated as described above. Even though the HAT knockdown significantly reduced the acetylation level of histone H3 as predicted, the addition of the two compounds enhanced the acetylation of the chromosome proteins and the acetylation of K56 present in the H3 globular region, to the level similar to that in control cells. This suggested that the chromosome protein-selective acetylation by the chromosome-localizable catalyst and the chromosome-localizable acetylating agent was not dependent on endogenous acetyltransferases (Fig. 5A, B). Hereinafter, this system to which 8DMAP and 3NMD-8R are added will be referred to as "synthetic chromosome acetylation SynCAc (synthetic chromatin acetylation by artificial catalyst)."

It has been known that a histone deacetylase (HDAC) inhibitor enhances the acetylations of histones and various proteins, and selectively exhibits a growth suppressing effect against some cancer cells. Hence, it is known to function as an anticancer agent (Marks, P. A. Oncogene 2007, 26, 1351-1356). MCF7 cells, HeLa cells, and hTERT RPE-1 (hereinafter, RPE-1) cells which are normal cells were treated with an HDAC inhibitor (SAHA). As a result, in any of the cell types, the acetylations of lysines in the tail regions of histones H3 and H4 were significantly enhanced; in the cytoplasms, the tubulin acetylation was significantly enhanced (Fig. 6). On the other hand, there was no change in the acetylation of K56 present in the H3 globular region (Fig. 6). A comparison was made among the same three cell types with the "synthetic chromosome acetylation SynCAc." As a result, chromosome protein- and histone H3 globular region- selective acetylation enhancements were observed in any of the cell types (Fig. 6). This result suggested that the "synthetic chromosome acetylation SynCAc" enhanced the acetylations of the histones and the chromosome proteins in a different pattern from that of the HDAC inhibitor, and that the reactions took place independently of the cell type.

Conceivably, there was a possibility that the "synthetic chromosome acetylation SynCAc" exhibited a cancer cell selectivity like the HDAC inhibitor. To examine the possibility, the cancer cell selectivity of the "synthetic chromosome acetylation SynCAc" was examined using the MCF7 cells which are cancer cells and RPE-1 cells which are normal cells. The ratios in the cell cycle were examined 15 hours, 18 hours, and 22 hours after the two compounds were administered to the MCF7 cells. Asaresult, it was found out that the ratio of the G1 phase was increased over time (Fig. 7A). Interestingly, such a phenotype as the Gl phase arrest was not observed at all when the two compounds were administered to the RPE-1 cells (Fig. 7B) . It has been known that the Gl phase arrest is controlled by a tumor suppressor gene p53 (Hartwell, L. H. & Kastan, M. B. Science 1994, 266, 1821-1828). To examine whether the Gl phase arrest by the "synthetic chromosome acetylation SynCAc" was dependent on p53, p53 was knocked down by the RNAi method (Fig. 7C). When the two compounds were administered to the MCF7 cells whose p53 had been knocked down, the Gl phase arrest did not occur. This revealed that the Gl phase arrest by the "synthetic chromosome acetylation SynCAc" was dependent on p53 (Fig. 7D).

The above result suggested a possibility that the "synthetic chromosome acetylation" activated p53. To examine this possibility, the P53 mRNA level was examined by the RT-qPCR method, and the protein level was examined by the western blotting method. As a result, the "synthetic chromosome acetylation SynCAc" increased the p53 mRNA and protein levels in the MCF7 cells, but such increases were not observed in the RPE-1 cells (Fig. 8A, B). p53 is known to cause the Gl phase arrest in the cell cycle by promoting the transcription of a cyclin-dependent kinase inhibiting factor p21 (CIP1/WAF1) downstream of p53 (Hartwell, L. H. & Kastan, M. B. Science 1994, 266, 1821-1828) . Moreover, it is known that increasing the expression of another cyclin-dependent kinase inhibiting factor p27 (KIP1) also causes the Gl phase arrest in the cell cycle, although whether it is under the control of p53 is not clear (Hartwell, L. H. & Kastan, M. B. Science 1994, 266, 1821-1828, Toyoshima, H. & Hunter, T. Cell 1994, 78, 67-74). Hence, next, the change in mRNA levels of p21 and p27 was examined by the RT-qPCR method, and the protein levels were examined by the western blotting method. As a result, the "synthetic chromosome acetylation" increased the mRNA and protein levels of p21 and p27 in the MCF7 cells, but such increases were not observed in the RPE-1 cells (Fig. 8B, C). Further, the increases were dependent on p53 (Fig. 8D). Note that the p21 protein level was investigated in other cancer cells and normal cells, too. As a result, the protein level was increased in LoVo cells (colorectal adenocarcinoma), RKO cells (colorectal cancer), andHT1080 cells (fibrosarcoma) which are cancer cells, but such an increase was not observed in MRC-5 cells which are normal cells.

On the basis of the above results, it was made possible to cause the endogenous acetyltransferase-independent "synthetic chromosome acetylation SynCAc" in cultured human cells by using the combination of the chromosome-localizable catalyst 8DMAP and the chromosome-localizable acetylating agent 3NMD-8R developed by the present inventors, and to promote the cell cycle arrest dependent on the tumor suppressor gene p53 in human cancer cells (Fig. 9).

### (Example 2) Chromosome Protein Malonylation with Chromosome-Localizable Malonylating Agent and Chromosome-Localizable Nucleophilic Catalyst

A reconstituted nucleosome (0.346 µM: in terms of DNA) and a cytoplasm extract extracted from HeLa cells were mixed in a 20 mM solution of Tris-HCl (pH 7.5), and the catalyst L-8DMAP and a malonylation donor 3Mal-BA-8R were added thereto and allowed to react at room temperature for 3 hours. The resultant was separated on an SDS-PAGE gel, and a malonylated protein was detected by the western blotting method. For comparison, a protein acetylated by the catalyst L-8DMAP and an acetylation donor 3NMD-8R was also detected similarly. As primary antibodies in the western blotting methods, Pan Anti-Malonyllysine Antibody (PTM-901, PTM Biolabs) was used for the malonylated protein detection, and Acetylated-Lysine antibody (#9441, Cell Signaling) was used for the acetylated protein detection.

As a result, the malonylation of lysines of histones H3 and H4 with the combination of L-8DMAP and 3Mal-BA-8R was detected (right in Fig. 10). With the combination of L-8DMAP and 3NMD-8R also, the acetylation of lysines of histones H3 and H4 was detected in consistent with the above experimental results (left in Fig. 10).

### [Industrial Applicability]

The artificial catalyst system based on a concept of catalysis medicine is applicable to various reactions in which *in vivo* enzymes are involved. Particularly, in a case where a loss or inactivation of an *in vivo* enzyme is involved in a particular disease, the disease can be treated with the artificial catalyst system targeting the enzyme. Therefore, the present invention can widely contribute to the medical field.

The artificial catalyst system using a combination of a chromosome-localizable nucleophilic catalyst and a chromosome-localizable acetylating agent is utilizable in treatments against diseases caused by a reduction in chromosome protein acetylation. Particularly, such an artificial catalyst system is very useful against diseases caused by histone acetyltransferase inactivation against which conventional histone deacetylase inhibitors can hardly exhibit efficacy.

The present invention has successfully established an artificial catalyst system based on a concept of catalysis medicine for the first time in the world, pioneering novel medical treatments which are fundamentally different from such a conventional medicinal concept as regulating endogenous enzyme functions.

### [Sequence Listing Free Text]

SEQ ID NOs: 1 to 10
   <223> siRNA sequence: Combined DNA/RNA Molecule
SEQ ID NOs: 11 to 20
   <223> Artificially synthesized primer sequence

## Claims

1. An agent for acylating a chromosome protein, the agent comprising a combination of the following compound (a) and the following compound (b):
(a) a compound having a chromosome-localizable activity and a nucleophilic catalytic activity, wherein the compound has a structure in which five or more molecules of 4-dimethylaminopyridine are linked with linkers and in which the carbon of a methyl group or position 3 or 5 of the pyridine in each 4-dimethylaminopyridine is linked to the linker; and
(b) a compound having a chromosome-localizable activity and a lysine acylating activity, wherein the compound has a structure in which an acylating agent and a cationic compound are linked, wherein said acylating agent is N-methoxydiacetamide or 4-malonyloxybenzamide and said cationic compound is a cationic polymer which is polyarginine.

2. The agent according to claim 1, wherein the compound (a) has any one of the following structures: 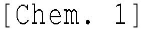 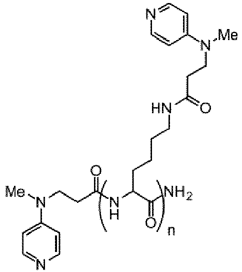 (n represents a natural number within such a range that the compound has the chromosome-localizable activity and the nucleophilic catalytic activity); and 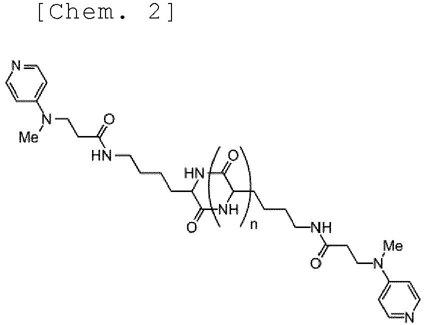 (n represents a natural number within such a range that the compound has the chromosome-localizable activity and the nucleophilic catalytic activity).

3. The agent according to claim 1, wherein:
(i) the acylating agent and the cationic compound in the compound (b) are linked with a linker; and/or
(ii) the compound (b) has the following structure: 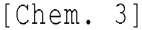 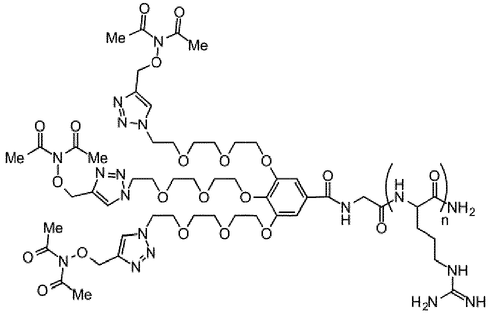 (n represents a natural number within such a range that the compound has the chromosome-localizable activity and a lysine acetylating activity); and/or
(iii) the compound (b) has the following structure: 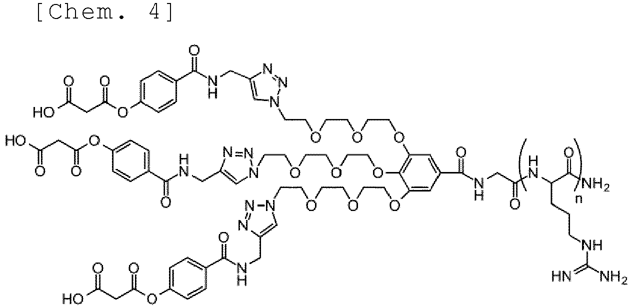 (n represents a natural number within such a range that the compound has the chromosome-localizable activity and a lysine malonylating activity).

4. A compound having a chromosome-localizable activity and a nucleophilic catalytic activity, wherein the compound has a structure in which five or more molecules of 4-dimethylaminopyridine are linked with linkers and in which the carbon of a methyl group or position 3 or 5 of the pyridine in each 4-dimethylaminopyridine is linked to the linker.

5. The compound according to claim 4, which has any one of the following structures: 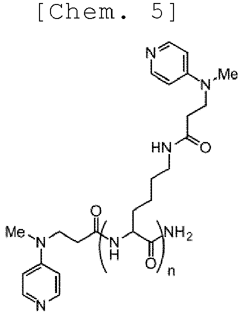 (n represents a natural number within such a range that the compound has the chromosome-localizable activity and the nucleophilic catalytic activity); and 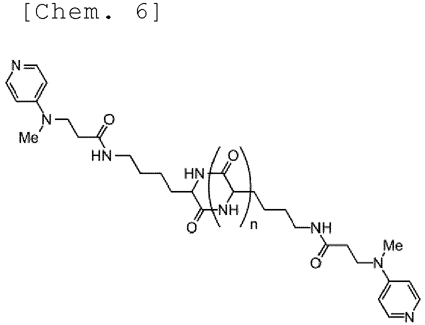 (n represents a natural number within such a range that the compound has the chromosome-localizable activity and the nucleophilic catalytic activity).

6. A compound having a chromosome-localizable activity and a lysine acylating activity, wherein the compound has a structure in which an acylating agent and a cationic compound are linked, wherein said acylating agent is N-methoxydiacetamide or 4-malonyloxybenzamide and said cationic compound is a cationic polymer which is polyarginine.

7. The compound according to claim 6:
(i) wherein the acylating agent and the cationic compound are linked with a linker; and/or
(ii) the compound has the following structure: 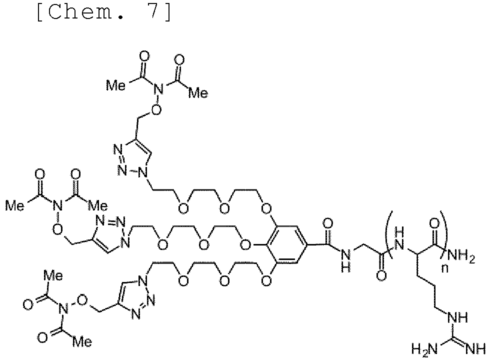 (n represents a natural number within such a range that the compound has the chromosome-localizable activity and a lysine acetylating activity); and/or
(iii) the compound has the following structure: 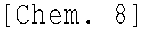 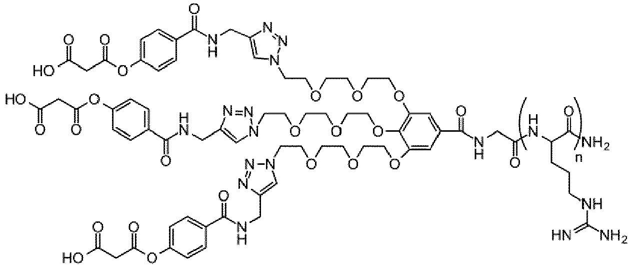 (n represents a natural number within such a range that the compound has the chromosome-localizable activity and a lysine malonylating activity).

8. An agent for use in a method of treatment by inducing an expression of any one of p53, p21, and p27 in a cancer cell, the agent comprising a combination of the compound according to claim 4 or claim 5 and the compound according to claim 6 or claim 7(i).

9. An agent for use in treating a disease caused by a reduction in chromosome protein acetylation, the agent comprising a combination of the compound according to claim 4 or claim 5 and the compound according to claim 6 or claim 7 (ii) .

10. The agent for use according to claim 9, wherein the disease caused by a reduction in chromosome protein acetylation is a cancer.

## Patentansprüche

1. Mittel zum Acylierung eines Chromosomenproteins, das Mittel umfassend eine Kombination der folgenden Verbindung (a) und der folgenden Verbindung (b):
(a) eine Verbindung, die eine chromosomenlokalisierbare Aktivität und eine nukleophile katalytische Aktivität aufweist, wobei die Verbindung eine Struktur aufweist, in der fünf oder mehrere Moleküle von 4-Dimethylaminopyridin mit Linkern verbunden sind und in der der Kohlenstoff einer Methylgruppe oder Position 3 oder 5 des Pyridins in jedem 4-Dimethylaminopyridin an den Linker gebunden ist; und
(b) eine Verbindung, die eine chromosomenlokalisierbare Aktivität und eine Lysin-acylierende Aktivität aufweist, wobei die Verbindung eine Struktur aufweist, in der ein Acylierungsmittel und eine kationische Verbindung verbunden sind, wobei das Acylierungsmittel N-Methoxydiacetamid oder 4-Malonyloxybenzamid ist und die kationische Verbindung ein kationisches Polymer ist, das Polyarginin ist.

2. Mittel nach Anspruch 1, wobei die Verbindung (a) eine der folgenden Strukturen aufweist: 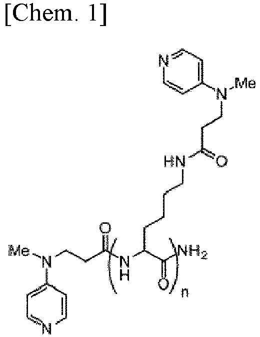 (n stellt eine natürliche Zahl innerhalb eines solchen Bereichs dar, dass die Verbindung die chromosomenlokalisierbare Aktivität und die nukleophile katalytische Aktivität aufweist); und 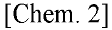 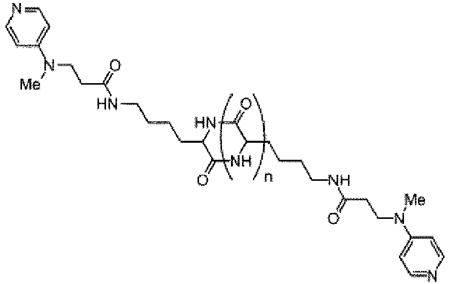 (n stellt eine natürliche Zahl innerhalb eines solchen Bereichs dar, dass die Verbindung die chromosomenlokalisierbare Aktivität und die nukleophile katalytische Aktivität aufweist).

3. Mittel nach Anspruch 1, wobei:
(i) das Acylierungsmittel und die kationische Verbindung in der Verbindung (b) mit einem Linker verbunden sind; und/oder
(ii) die Verbindung (b) die folgende Struktur aufweist: 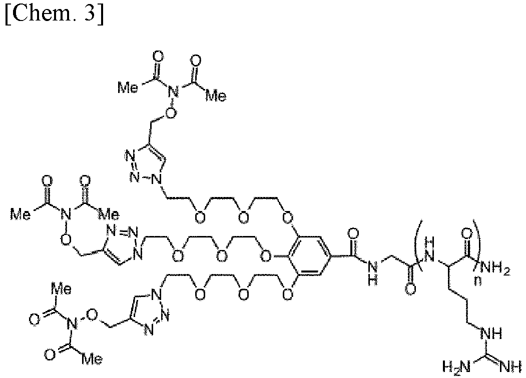 (n stellt eine natürliche Zahl innerhalb eines solchen Bereichs dar, dass die Verbindung die chromosomenlokalisierende Aktivität und eine Lysin-acetylierende Aktivität aufweist); und/oder
(iii) die Verbindung (b) die folgende Struktur aufweist: 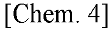 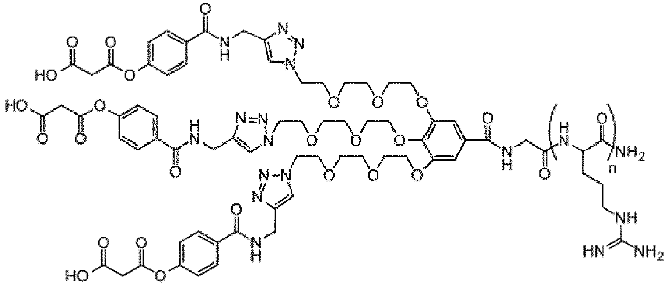 (n stellt eine natürliche Zahl innerhalb eines solchen Bereichs dar, dass die Verbindung die chromosomenlokalisierende Aktivität und eine Lysin-malonylierende Aktivität aufweist).

4. Verbindung, die eine chromosomenlokalisierbare Aktivität und eine nukleophile katalytischen Aktivität aufweist, wobei die Verbindung eine Struktur aufweist, in der fünf oder mehrere Moleküle von 4-Dimethylaminopyridin mit Linkern verbunden sind und in der der Kohlenstoff einer Methylgruppe oder Position 3 oder 5 des Pyridins in jedem 4-Dimethylaminopyridin an den Linker gebunden ist.

5. Verbindung nach Anspruch 4, die eine der folgenden Strukturen aufweist: 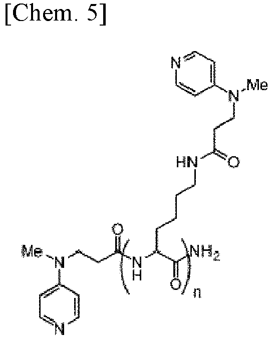 (n stellt eine natürliche Zahl innerhalb eines solchen Bereichs dar, dass die Verbindung die chromosomenlokalisierbare Aktivität und die nukleophile katalytische Aktivität aufweist); und 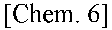 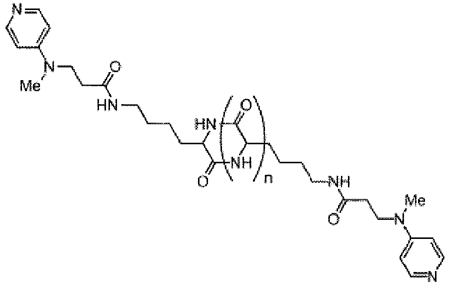 (n stellt eine natürliche Zahl innerhalb eines solchen Bereichs dar, dass die Verbindung die chromosomenlokalisierbare Aktivität und die nukleophile katalytische Aktivität aufweist).

6. Verbindung, die eine chromosomenlokalisierbare Aktivität und eine Lysin-acylierende Aktivität aufweist, wobei die Verbindung eine Struktur aufweist, in der ein Acylierungsmittel und eine kationische Verbindung verbunden sind, wobei das Acylierungsmittel N-Methoxydiacetamid oder 4-Malonyloxybenzamid ist und die kationische Verbindung ein kationisches Polymer ist, das Polyarginin ist.

7. Verbindung nach Anspruch 6:
(i) wobei das Acylierungsmittel und die kationische Verbindung mit einem Linker verbunden sind; und/oder
(ii) die Verbindung die folgende Struktur aufweist: 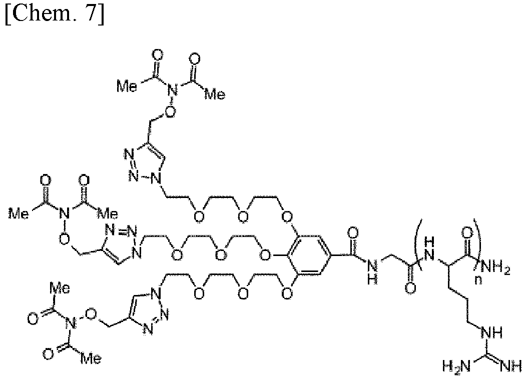 (n stellt eine natürliche Zahl innerhalb eines solchen Bereichs dar, dass die Verbindung die chromosomenlokalisierende Aktivität und eine Lysin-acetylierende Aktivität aufweist); und/oder
(iii) die Verbindung die folgende Struktur aufweist: 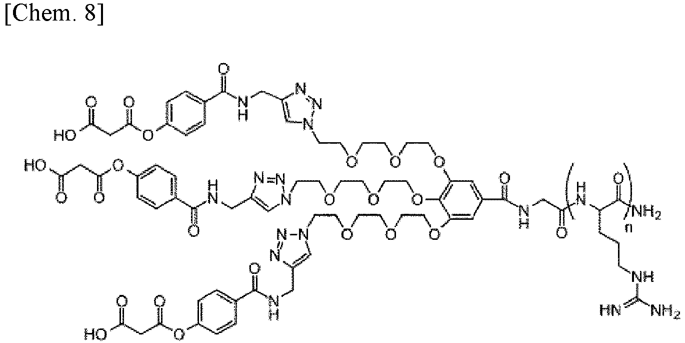 (n stellt eine natürliche Zahl innerhalb eines solchen Bereichs dar, dass die Verbindung die chromosomenlokalisierende Aktivität und eine Lysin-malonylierende Aktivität aufweist).

8. Mittel zur Verwendung in einem Behandlungsverfahren durch Induzieren einer Expression von einem von p53, p21 und p27 in einer Krebszelle, das Mittel umfassend eine Kombination der Verbindung nach Anspruch 4 oder 5 und der Verbindung nach Anspruch 6 oder 7 (i).

9. Mittel zur Verwendung beim Behandeln einer Krankheit, die durch eine Verringerung von Chromosomenprotein-Acetylierung verursacht wird, das Mittel umfassend eine Kombination der Verbindung nach Anspruch 4 oder Anspruch 5 und der Verbindung nach Anspruch 6 oder Anspruch 7 (ii).

10. Mittel zur Verwendung nach Anspruch 9, wobei die Krankheit, die durch eine Verringerung von Chromosomenprotein-Acetylierung verursacht wird, ein Krebs ist.

## Revendications

1. Agent d'acylation d'une protéine chromosomique, l'agent comprenant une combinaison du composé (a) suivant et du composé (b) suivant :
(a) un composé ayant une activité chromosomique localisable et une activité catalytique nucléophile, dans lequel le composé a une structure dans laquelle cinq ou plusieurs molécules de 4-diméthylaminopyridine sont liées à des lieurs et dans lequel le carbone d'un groupe méthyle ou en position 3 ou 5 de la pyridine dans chaque 4-diméthylaminopyridine est lié au lieur ; et
(b) un composé ayant une activité chromosomique localisable et une activité d'acylation de la lysine, dans lequel le composé a une structure dans laquelle un agent d'acylation et un composé cationique sont liés, dans lequel ledit agent d'acylation est le N-méthoxydiacétamide ou le 4-malonyloxybenzamide et ledit composé cationique est un polymère cationique qui est la polyarginine.

2. Agent selon la revendication 1, dans lequel le composé (a) a l'une quelconque des structures suivantes : 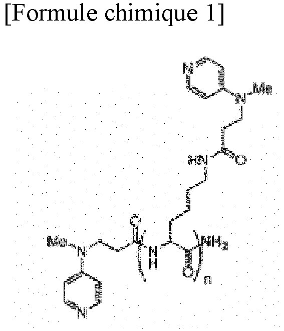 (n représente un nombre naturel dans une plage de telle sorte que le composé possède l'activité chromosomique localisable et l'activité catalytique nucléophile) ; et 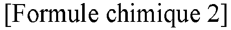 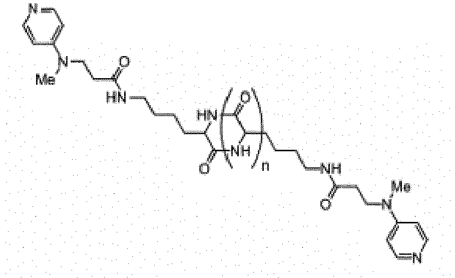 (n représente un nombre naturel dans une plage de telle sorte que le composé possède l'activité chromosomique localisable et l'activité catalytique nucléophile).

3. Agent selon la revendication 1, dans lequel :
(i) l'agent d'acylation et le composé cationique dans le composé (b) sont liés à un lieur ; et / ou
(ii) le composé (b) a la structure suivante : 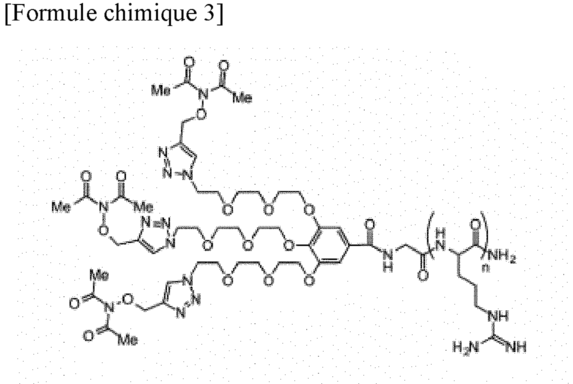 (n représente un nombre naturel dans une plage de telle sorte que le composé possède l'activité chromosomique localisable et une activité d'acétylation de la lysine) ; et / ou
(iii) le composé (b) a la structure suivante : 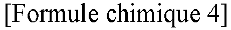 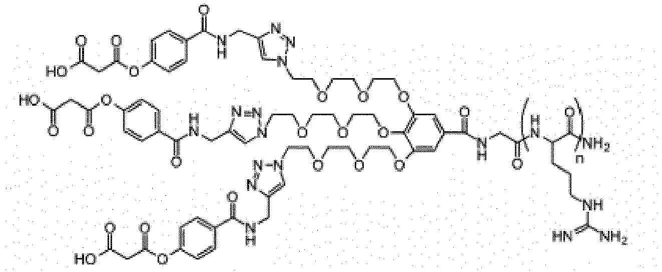 (n représente un nombre naturel dans une plage de telle sorte que le composé possède l'activité chromosomique localisable et une activité de malonylation de la lysine).

4. Composé ayant une activité chromosomique localisable et une activité catalytique nucléophile, dans lequel le composé a une structure dans laquelle cinq ou plusieurs molécules de 4-diméthylaminopyridine sont liées à des lieurs et dans lequel le carbone d'un groupe méthyle ou en position 3 ou 5 de la pyridine dans chaque 4-diméthylaminopyridine est lié au lieur.

5. Composé selon la revendication 4, qui a l'une quelconque des structures suivantes : 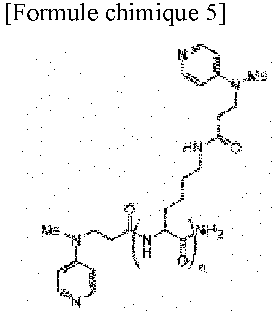 (n représente un nombre naturel dans une plage de telle sorte que le composé possède l'activité chromosomique localisable et l'activité catalytique nucléophile) ; et 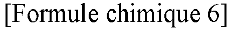 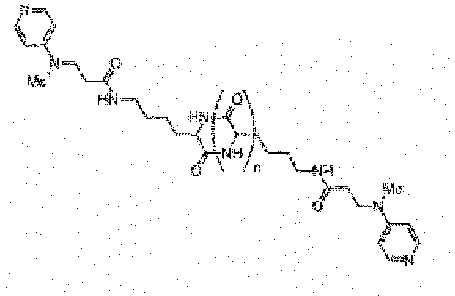 (n représente un nombre naturel dans une plage de telle sorte que le composé possède l'activité chromosomique localisable et l'activité catalytique nucléophile).

6. Composé ayant une activité chromosomique localisable et une activité d'acylation de la lysine, dans lequel le composé a une structure dans laquelle un agent d'acylation et un composé cationique sont liés, dans lequel ledit agent d'acylation est le N-méthoxydiacétamide ou le 4-malonyloxybenzamide et ledit composé cationique est un polymère cationique qui est la polyarginine.

7. Composé selon la revendication 6 :
(i) dans lequel l'agent d'acylation et le composé cationique sont liés à un lieur ; et / ou
(ii) le composé a la structure suivante : 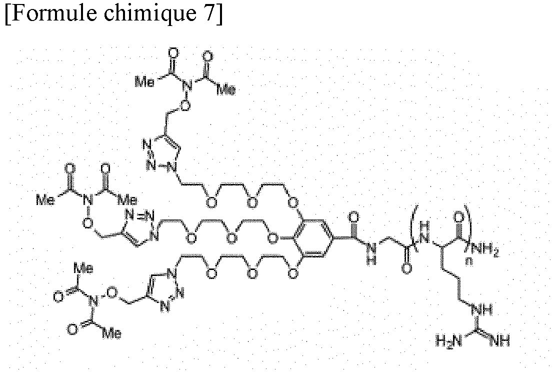 (n représente un nombre naturel dans une plage de telle sorte que le composé possède l'activité chromosomique localisable et une activité d'acétylation de la lysine) ; et / ou
(iii) le composé a la structure suivante : 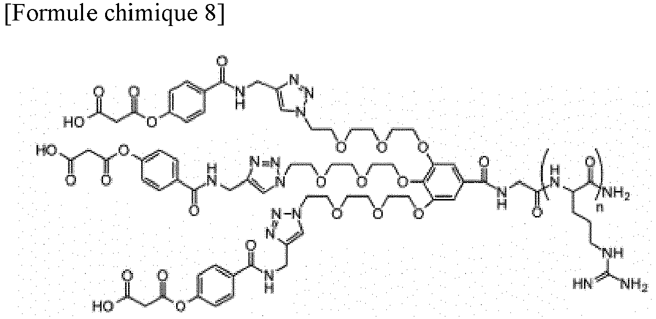 (n représente un nombre naturel dans une plage de telle sorte que le composé possède l'activité chromosomique localisable et une activité de malonylation de la lysine).

8. Agent à utiliser dans une méthode de traitement en induisant une expression de l'une quelconque parmi p53, p21 et p27 dans une cellule cancéreuse, l'agent comprenant une combinaison du composé selon la revendication 4 ou la revendication 5 et le composé selon la revendication 6 ou la revendication 7 (i).

9. Agent à utiliser dans le traitement d'une maladie provoquée par une réduction de l'acétylation des protéines chromosomiques, l'agent comprenant une combinaison du composé selon la revendication 4 ou la revendication 5 et le composé selon la revendication 6 ou la revendication 7 (ii).

10. Agent à utiliser selon la revendication 9, dans lequel la maladie provoquée par une réduction de l'acétylation des protéines chromosomiques est un cancer.
